(19) Europäisches Patentamt
European Patent Office
Office européen des brevets

(11) EP 0 880 524 B1

(12) **EUROPÄISCHE PATENTSCHRIFT**

(45) Veröffentlichungstag und Bekanntmachung des Hinweises auf die Patenterteilung:
**19.04.2000 Patentblatt 2000/16**

(21) Anmeldenummer: **97933683.1**

(22) Anmeldetag: **17.07.1997**

(51) Int Cl.[7]: **C07D 487/14**, A61K 31/505
// (C07D487/14, 249:00, 239:00, 235:00)

(86) Internationale Anmeldenummer:
**PCT/EP97/03841**

(87) Internationale Veröffentlichungsnummer:
**WO 98/03511 (29.01.1998 Gazette 1998/04)**

(54) **ADENOSIN-ANTAGONISTEN, VERFAHREN ZU IHRER HERSTELLUNG UND IHRE VERWENDUNG ALS ARZNEIMITTEL**

ADENOSIN ANTAGONISTS, METHOD OF PREPARING THEM AND THEIR USE AS DRUGS

ANTAGONISTES D'ADENOSINE, PROCEDE POUR LES PREPARER ET LEUR UTILISATION COMME MEDICAMENTS

(84) Benannte Vertragsstaaten:
**AT BE CH DE DK ES FI FR GB GR IE IT LI LU MC NL PT SE**
Benannte Erstreckungsstaaten:
**LT LV RO SI**

(30) Priorität: **20.07.1996 DE 19629378**

(43) Veröffentlichungstag der Anmeldung:
**02.12.1998 Patentblatt 1998/49**

(60) Teilanmeldung: **99119410.1 / 0 978 517**

(73) Patentinhaber:
- **Boehringer Ingelheim Pharma KG**
**55216 Ingelheim am Rhein (DE)**
Benannte Vertragsstaaten:
**BE CH DE DK ES FI FR GR IT LI LU MC NL PT SE AT**
- **Boehringer Ingelheim International GmbH**
**55216 Ingelheim (DE)**
Benannte Vertragsstaaten:
**GB IE**

(72) Erfinder:
- **KÜFNER-MÜHL, Ulrike**
**D-55218 Ingelheim am Rhein (DE)**
- **KUMMER, Werner**
**D-55218 Ingelheim am Rhein (DE)**
- **POHL, Gerald**
**D-55435 Gau-Algesheim (DE)**
- **GAIDA, Wolfram**
**D-55218 Ingelheim am Rhein (DE)**
- **LEHR, Erich**
**D-55425 Waldalgesheim (DE)**
- **MIERAU, Joachim**
**D-55127 Mainz am Rhein (DE)**
- **WEISER, Thomas**
**D-55268 Nieder-Olm (DE)**

(74) Vertreter: **Laudien, Dieter, Dr. et al**
**Boehringer Ingelheim GmbH**
**Abteilung Patente**
**55216 Ingelheim (DE)**

(56) Entgegenhaltungen:
**EP-A- 0 390 111** **WO-A-95/01356**

- **CHEMICAL ABSTRACTS, vol. 124, no. 23, 1996 Columbus, Ohio, US; abstract no. 317171p, NAGAMATSU ET AL.: "Preparation of triazolopurinone derivatives as tricyclic bases for nucleic acid" Seite 1221; XP002046862 & JP 8 003 169 A (YAMASA) 09 Januar 1996**
- **Chem. Ber. 97 (1964), Seiten 1373-1382**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft neue Adenosin-Antagonisten, Verfahren zu ihrer Herstellung und ihre Verwendung als Arzneimittel.

Verbindungen mit adenosinantagonistischen Eigenschaften sind aus dem Stand der Technik bekannt. So offenbart die WO 95/01356 heterocyclische Analoga des 1,2,4-Triazolo[1,5-c]pyrimidins, welche durch eine antagonistische Aktivität zum Adenosin-$A_2$-Rezeptor gekennzeichnet sind. EP-A-390 111 beschreibt Imidazo[1,2-c]pyrazol [4,5-e]pyrimidine sowie Diimidazo[1,2-c:4',5'-e]pyrimidine die eine Affinität zum Adenosin-$A_1$- und/oder Adenosin-$A_2$-Rezeptor aufweisen. Durch Tenor et al. (Chem. Ber. 97 (1964), Seiten 1373-1378) wurde eine neue Klasse heterocyclischer Verbindungen, die Imidazo[4,5-e]-s-triazolo[1,5-a]pyrimidin-5-one, durch Synthese zweier exemplarischer Verbindungen erstmals beschrieben. Eine potentielle Wirksamkeit als Arzneimittel wurde bezüglich besagter exemplarischer Verbindungen, dem 7-Methyl-2-ethyl-imidazo[4,5-e]-s-triazolo[1,5-a]pyrimidin-5-one sowie dem 7-Methyl-2-propyl-imidazo[4,5-e]-s-triazolo[1,5-a]pyrimidin-5-one, nicht offenbart.

Überraschenderweise wurde gefunden, daß Derivate der durch Tenor et al. offenbarten Imidazo[4,5-e]-s-triazolo[1,5-a]pyrimidin-5-one eine Affinität zu Adenosin-Rezeptoren aufweisen und somit eine neue Klasse von Adenosinantagonisten darstellen.

Die vorliegende Erfindung betrifft dementsprechend Verbindungen mit adenosinantagonistischen Eigenschaften, die durch folgende Reaktionsschritte erhältlich sind:

a) Umsetzung von 0,1 Mol eines Triazols der allgemeinen Formel (3)

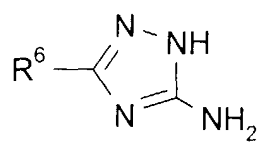

(3)

worin

$R^6$ Wasserstoff, Methyl, Ethyl, n-Propyl, i-Propyl, n-Butyl, tert.-Butyl, Phenyl, Benzyl, Cyclopentyl, 2-Furyl, 3-Pyridylmethyl, Cyclohexylmethyl, 2-Phenylethyl, (4-MeO-Ph)-O-$CH_2$-, (4-MeO-Ph)-$CH_2$-O-$CH_2$-, (4-$PhCH_2$O-Ph)-$CH_2$-, 4-F-Ph-$CH_2$- oder 3,4-F-Ph-$CH_2$- bedeuten können,

mit 0,13 Mol Cyanessigsäureethylester in Gegenwart von 0,1 Mol Natriumethylat in absolutem Ethanol unter Rückfluß über einen Zeitraum von 4-6 h zu dem entsprechenden Triazolopyrimidin, welches nach Abkühlen des Reaktionsgemisches sowie anschließendem Versetzen mit Wasser und Ansäuern isoliert wird

b) anschließende Umsetzung von 66mMol des so erhaltenen Triazolopyrimidins bei Raumtemperatur mit 76mMol eines Alkylhalogenids des Typs R'-X, wobei R' für den Rest $R^4$ steht, wobei $R^4$ Methyl, Ethyl, n-Propyl, n-Butyl, n-Pentyl oder Benzyl bedeutet, in wasserfreiem Dimethylformamid in Gegenwart von 76mMol Kaliumcarbonat über einen Zeitraum von 1-2 Tagen zu dem entsprechenden alkylierten Triazolopyrimidin, das nach dessen Isolierung gegebenenfalls einer chromatographischen Reinigung unterworfen wird,

c) Umsetzung von 10mMol des aus Schritt b) resultierenden Alkyltriazolopyrimidins mit 20 mMol Isoamylnitrit bei einer Temperatur von <0°C in wasserfreiem Dimethylformamid über einen Zeitraum von 4-24 h zu der entsprechenden Nitrosoverbindung, die isoliert wird,

d1) Suspendieren von 12,7 mMol eines gemäß Schritt c) erhaltenen Nitrosoderivats in Wasser, Zusetzen von 4,9 g $Na_2S_2O_4$, Erhitzen der Suspension auf 80°C, Zugabe von 15 ml 50%iger $H_2SO_4$ und Kochen der Mischung unter Rückfluß bis zum vollständigen Umsatz, wodurch nach Abkühlen das Produkt entweder dirket als ausfallendes Semisulfat oder, nach Neutralisieren mit 30%-iger Natronlauge, als ausgefallene freie Base isoliert werden kann,

oder

d2) Lösen von 11,5 mMol eines gemäß Schritt c) erhaltenen Nitrosoderivats in einer Mischung aus 175 ml 25%-igem wässrigen Ammoniak und 36 ml Ethanol und Umsetzung mit einer Lösung von 6,1 g $Na_2S_2O_4$ in 57 ml Wasser bei Raumtemperatur über einen Zeitraum von 1,5-3 h, Abfiltrieren des resultierenden Diamins,

e) Versetzen von 30 mMol eines gemäß Schritt d1) oder gemäß Schritt d2) erhaltenen, in wasserfreiem Diemthylformamid suspendierten, Diamins mit 45 mMol Dimethylaminopyridin (im Falle von Semisulfaten mit 78 mMol) und Rühren der Mischung über einen Zeitraum von 30 Minuten bei Raumtemperatur, Abkühlen des Reaktionsgemisches auf eine Temperatur von 5-10°C und Zugabe von in 16 ml wasserfreiem Dimethylformamid gelösten 39 mMol eines Carbonsäurechlorids des Typs $R^2$-COCl,
wobei

$R^2$ Methyl, Ethyl, n-Propyl, tert-Butyl, Phenyl, Benzyl, 2-Furyl, 2-Pyridyl, 3-Pyridyl, 4-Pyridyl, 3-Tetrahydrofuranyl, 4-Tetrahydropyranyl, Cyclopentyl, Cyclohexyl, 4-F-Ph-$CH_2$-, Adamantan-1-yl oder Noradamantan-3-yl bedeuten,

sowie 2-stündiges Rühren bei 10°C und anschließendes Rühren über Nacht bei Raumtemperatur, woraus die entsprechenden Amide resultieren, die nach Aufarbeitung und Reinigung durch Kristallisation oder Chromatographie isoliert werden,

f) Cyclisieren von 14,4 mMol der in Schritt e) erhaltenen Carbonsäureamiderivate durch Erhitzen der Amide in einem Lösemittelgemisch aus 75,5 ml Wasser und 37,8 ml Ethanol nach Zugabe von 17,4 ml 50%iger NaOH und 4,82 g $Ca(OH)_2$ über einen Zeitraum von 24 h bei Rückflußtemperatur und Isolieren der entsprechenden Cyclisierungsprodukte nach Abkühlen und Ansäuern des Reaktionsgemisches sowie gegebenenfalls Reinigung durch Umkristallisation oder Chromatographie.

Gegebenenfalls können die erfindungsgemäßen Verbindungen, insbesondere für die pharmazeutische Anwendung, in ihre physiologisch verträglichen Salze mit einer anorganischen oder organischen Säure, überführt werden. Als Säuren kommen hierfür beispielsweise Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure in Betracht. Ferner können Mischungen der vorgenannten Säuren eingesetzt werden.

Generell können Adenosin-Antagonisten in den Fällen eine therapeutisch nutzbare Wirkung entfalten, in denen Krankheiten oder pathologische Situationen mit einer Aktivierung von Adenosin-Rezeptoren verbunden sind.

Adenosin ist ein endogener Neuromodulator mit überwiegend hemmenden (inhibitorischen) Wirkungen im ZNS, im Herzen, in den Nieren und anderen Organen. Die Effekte von Adenosin werden über mindestens drei Rezeptor-Subtypen vermittelt: Adenosin $A_1$ -, $A_2$- und $A_3$- Rezeptoren.

Im ZNS entfaltet Adenosin inhibitorische Wirkungen vorwiegend über die Aktivierung von $A_1$ -Rezeptoren: praesynaptisch durch Hemmung der synaptischen Übertragung (Hemmung der Freisetzung von Neurotransmittern wie Acetylcholin, Dopamin, Noradrenalin, Serotonin, Glutamat u.a.), postsynaptisch durch Hemmung der neuronalen Aktivität.

$A_1$-Antagonisten heben die inhibitorischen Wirkungen von Adenosin auf und fördern die neuronale Transmission und die neuronale Aktivität.

$A_1$ Antagonisten sind deshalb von großem Interesse für die Therapie zentralnervöser degenerativer Erkrankungen wie senile Demenz vom Morbus Alzheimer Typ und altersassoziierte Störungen der Gedächtnis- und Lernleistungen.

Die Krankheit umfaßt neben der Vergeßlichkeit in der milden Form und der völligen Hilflosigkeit und absoluten Pflegebedürftigkeit bei der schwersten Form eine Reihe anderer Begleitsymptome wie Schlafstörungen, Moto-Koordinationsstörungen bis zum Bild eines Morbus Parkinson, ferner eine erhöhte Affektlabilität sowie auch depressive Symptome. Die Krankheit ist progredient und kann zum Tode führen. Die bisherige Therapie ist unbefriedigend. Spezifische Therapeutika fehlen bis jetzt vollständig. Therapieversuche mit Acetylcholinesterase-Inhibitoren zeigen nur bei einem geringen Teil der Patienten eine Wirkung, sind jedoch mit einer hohen Nebenwirkungsrate verbunden.

Die Pathophysiologie des M. Alzheimer und SDAT ist charakterisiert durch eine schwere Beeinträchtigung des cholinergen Systems, jedoch sind auch andere Transmittersysteme betroffen. Durch den Verlust praesynaptischer cholinerger und anderer Neurone und der daraus resultierenden mangelnden Bereitstellung von Neurotransmittern ist die neuronale Übertragung und die neuronale Aktivität in den für Lernen und Gedächtnis essentiellen Hirnarealen empfindlich vermindert.

Selektive Adenosin $A_1$-Rezeptor Antagonisten fördern die neuronale Transmission durch vermehrte Bereitstellung von Neurotransmittern, erhöhen die Erregbarkeit postsynaptischer Neurone und können damit der Krankheit symptomatisch entgegenwirken.

Die hohe Rezeptoraffinität und -Selektivität einiger der beanspruchten Verbindungen sollte es erlauben, M.

Alzheimer und SDAT mit niedrigen Dosen zu therapieren, so daß kaum mit Nebenwirkungen zu rechnen ist, die nicht auf die Blockade von $A_1$-Rezeptoren zurückzuführen sind.

Eine weitere Indikation für zentralwirksame Adenosin-$A_1$-Antagonisten ist die Depression. Der Therapieerfolg antidepressiver Substanzen scheint mit einer Aufregulation von $A_1$-Rezeptoren verbunden zu sein. $A_1$-Antagonisten können zur Aufregulierung von Adenosin-$A_1$-Rezeptoren führen und somit einen neuen Therapieansatz zur Behandlung von depressiven Patienten bieten.

Auch zur Therapie peripherer Indikationen können Adenosinantagonisten Verwendung finden.
Beispielsweise kann die Aktivierung von $A_1$-Rezeptoren in der Lunge zu einer Bronchokonstriktion führen. Selektive Adenosin $A_1$- Antagonisten relaxieren die tracheale glatte Muskulatur, bewirken eine Brochodilatation und können dadurch als Antiasthmamittel nützlich sein.

Wichtige Therapiefelder für Adenosin-Antagonisten sind ferner kardiovaskuläre Erkrankungen und Nierenerkrankungen.

Am Herzen entfaltet Adenosin über die Aktivierung von $A_1$-Rezeptoren eine Hemmung der elektrischen und kontraktilen Aktivität. Verbunden mit einer über $A_2$-Rezeptoren mediierten koronaren Vasodilatation wirkt Adenosin negativ chronotrop,-inotrop,-dromotrop, -bathmotrop, bradykard und erniedrigt das Herzminutenvolumen.

Adenosin $A_1$-Rezeptor-Antagonisten vermögen durch Ischämie und nachfolgende Reperfusion bedingte Schädigungen am Herzen und an der Lunge zu verhindern. Deshalb könnten Adenosinantagonisten zur Prävention oder frühen Behandlung von Ischämie-Reperfusions bedingten Schädigungen des Herzenz z.B. nach coronar Bypass-Chirurgie, Herztransplantation, Angioplastie oder thrombolytischer Therapie des Herzens und ähnlicher Eingriffe eingesetzt werden. Entsprechendes gilt für die Lunge.

An den Nieren bewirkt die Aktivierung von $A_1$-Rezeptoren eine Vasokonstriktion afferenter Arteriolen und dadurch bedingt einen Abfall des renalen Blutflusses und der glomerulären Filtration.

$A_1$ Antagonisten wirken an der Niere wie starke kaliumsparende Diuretika und können somit zur Nierenprotektion sowie zur Behandlung von Oedemen, Niereninsuffizienz und akutem Nierenversagen eingesetzt werden.

Aufgrund des Adenosin-Antagonismus am Herzen und der diuretischen Wirkung können $A_1$-Antagonisten bei verschiedenen kardiovaskulären Erkrankungen therapeutisch wirksam eingesetzt werden wie z.B. bei Herzinsuffizienz, Arrhytmien (Bradyarrhytmien) assoziiert mit Hypoxie oder Ischämie, Überleitungsstörungen, Hypertonie, Ascites bei Leberversagen (hepato-renales Syndrom) und als Analgetikum bei Durchblutungsstörungen.

Überraschenderweise zeigen einige der erfindungsgemäßen Verbindungen auch eine Affinität zum $A_3$-Adenosin-Rezeptor. $A_3$ Antagonisten hemmen die durch $A_3$-Rezeptor-Aktivierung bedingte Degranulation von Mastzellen und sind daher therapeutisch nützlich bei allen Krankheiten und pathologischen Situationen, die in Zusammenhang mit Mastzellen-Degranulation stehen: z.B. als antiinflammatorische Substanzen, bei Überempfindlichkeitsreaktionen wie z.B. Asthma, allergischer Rhinitis, Urticaria, bei myocardialer reperfusion injury, Scleroderma, Arthritis, Autoimmun-Krankheiten, entzündlichen Darmkrankheiten u.a..
Die zystische Fibrose - auch als Mukoviszidose bekannt - ist eine erbliche Stoffwechselstörung, hervorgerufen durch einen genetischen Defekt eines bestimmten Chromosoms. Durch eine vermehrte Produktion und erhöhte Viskosität des Sekrets der mukösen Drüsen in den Bronchien kann es zu schweren Komplikationen im Bereich der Atemwege kommen. Erste Untersuchungen haben gezeigt, daß $A_1$-Antagonisten den Efflux von Chloridionen z.B. bei CF PAC Zellen erhöhen. Ausgehend von diesen Befunden kann erwartet werden, daß bei Patienten, die an zystischer Fibrose (Mukovizidose) erkrankt sind, die erfindungsgemäßen Verbindungen den gestörten Elektrolythaushalt der Zellen regulieren und die Symptome der Erkrankung gemildert werden.

Die ermittelten $A_1$-Rezeptorbindungswerte wurden in Analogie zu Ensinger et al. in "Cloning and functional characterisation of human $A_1$ adenosine Receptor - Biochemical and Biophysical Communications, Vol 187, No. 2, 919-926, 1992" bestimmt und sind in Tabelle 9 zusammengefaßt.
Die in Tabelle 10 zusammengefassten $A_3$-Rezeptorbindungswerte wurden in Analogie zu Salvatore et al. "Molecular cloning and characterization of the human $A_3$-adenosine receptor" (Proc. Natl. Acad. Sci. USA 90, 10365-10369, 1993) ermittelt.

Die neuen Verbindungen können oral, transdermal, inhalativ oder parenteral verabreicht werden. Die erfindungsgemäßen Verbindungen liegen hierbei als aktive Bestandteile in üblichen Darreichungsformen vor, beispielsweise in
Zusammensetzungen, die im wesentlichen aus einem inerten pharmazeutischen Träger und einer effektiven Dosis des Wirkstoffs bestehen, wie beispielsweise Tabletten, Dragées, Kapseln, Oblaten, Pulver, Lösungen, Suspensionen, Emulsionen, Sirupe, Suppositorien, transdermale Systeme etc.. Eine wirksame Dosis der erfindungsgemäßen Verbindungen liegt bei einer oralen Anwendung zwischen 1 und 100, vorzugsweise zwischen 1 und 50, besonders bevorzugt zwischen 5-30 mg/Dosis, bei intravenöser oder intramuskulärer Anwendung zwischen 0,001 und 50, vorzugsweise zwischen 0,1 und 10 mg/Dosis. Für die Inhalation sind erfindungsgemäß Lösungen geeignet, die 0,01 bis 1,0, vorzugsweise 0,1 bis 0,5 % Wirkstoff enthalten. Für die inhalative Applikation ist die Verwendung von Pulvern bevorzugt. Gleichfalls ist es möglich, die erfindungsgemäßen Verbindungen als Infusionslösung, vorzugsweise in einer physiologischen Kochsalzlösung oder Nährsalzlösung einzusetzen.
Die erfindungsgemäßen Verbindungen können nach folgenden Verfahren hergestellt werden. In einer ersten Stufe wird Aminoguanidin (1) mit einem Carbonsäurederivat der allgemeinen Formel (2) zu einem Triazol der allgemeinen Formel (3) umgesetzt (Schema 1). Diese Reaktion kann gemäß den in J. Chem. Soc. 1929, 816; J. Org. Chem. 1926, 1729 oder Org. Synthesis 26, 11 veröffentlichten Vorschriften erfolgen.

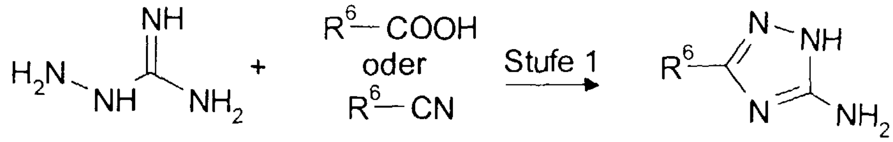


(1) (2) (3)

Schema 1:

Anschließend wird in einer zweiten Stufe das Triazol der allgemeinen Formel (3) mit Cyanessigsäurealkylester in einer Ringschlußreaktion unter alkalischen Bedingungen zu einem Triazolopyrimidin (4) umgesetzt.

Als Base kommen Alkali- oder Erdalkalialkoholate beispielsweise des Methanols, Ethanols, Isopropanols, n-, sec-, tert-Butylalkohols in Betracht. Geeignete Alkali- und Erdalkalimetalle sind beispielsweise Lithium, Natrium, Kalium, Magnesium, Calzium. Natriummethanolat, Natriumethanolat, Natriumisopropanolat und Kalium-tert-butylat sind als Base besonders bevorzugt. Weiterhin kommen erfindungsgemäß als Basen
Alkali- oder Erdalkalihydride in Betracht. Die Hydride des Natriums, Lithiums, Kaliums sowie des Magnesiums, Calziums sind bevorzugt. Geeignete inerte Lösungsmittel sind Dimethylformamid, Dimethylacetamid, Methylenchlorid, Tetrahydrofuran. Ferner können Alkali- oder Erdalkalihydroxide des Lithiums, Natriums, Kaliums sowie des Magnesiums, Calziums, bevorzugt jedoch Natriumhydroxid, Kaliumhydroxid, Lithiumhydroxid und Calziumhydroxid in alkoholischer oder wässriger Lösung zum Einsatz kommen.
Neben den Cyanessigsäurealkylestern kann gleichfalls Cyanessigsäure eingesetzt werden. Die so erhaltene Mischung wird über 0,5 bis 4 Stunden, bevorzugt 1 bis 2 Stunden, bei Raumtemperatur gerührt und anschließend mit einer Verbindung der allgemeinen Formel (3) versetzt und über 2 bis 12, bevorzugt 4 bis 6 Stunden gerührt, bevorzugt unter Rückfluß erhitzt. Das Reaktionsgemisch wird dann bei Raumtemperatur mit Wasser versetzt und sauer gestellt, anschließend wird der Feststoff abfiltriert, gewaschen und getrocknet. Geeignete Säuren sind beispielsweise Ameisensäure, Essigsäure oder mineralische Säuren, wie beispielsweise Salzsäure oder Schwefelsäure.

In einer dritten Stufe wird der Rest $R^4$ in das Triazolopyrimidin (4) eingeführt und man gelangt zu den alkylierten Verbindungen (5).

Hierzu wird eine Verbindung (4) mit der 5 bis 40-fachen, bevorzugt 10 bis 20-fachen Menge eines polaren Lösungsmittels, wie Dimethylformamid, Dimethylactamid, Methylenchlorid, Tetrahydrofuran, bevorzugt Dimethylformamid und besonders bevorzugt wasserfreies, gegebenenfalls absolutes Dimethylformamid gelöst. Die so erhaltene Lösung wird mit einer Base und einem entsprechenden Alkylierungsmittel versetzt. Als Base kommen die Alkali- oder die Erdalkalicarbonate des Lithiums, Natriums, Kaliums, Calziums wie Natriumcarbonat, Lithiumcarbonat, Kaliumcarbonat, Calziumcarbonat und bevorzugt Kaliumcarbonat in Betracht. Ferner können die Hydrogencarbonate des Lithiums, Natriums und Kaliums eingesetzt werden. Ferner sind die Alkali- oder Erdalkalihydroxide des Lithiums, Natriums, Kaliums, Magnesiums, Calziums, bevorzugt jedoch Natriumhydroxid, Kaliumhydroxid, Lithiumhydroxid und Calziumhydroxid in Alkoholen oder Wasser einsetzbar. Als weitere Basen kommen die in Stufe 2 bereits aufgeführten Alkoholate der Alkali- und Erdalkalimetalle in Betracht. Weiterhin können die zuvor genannten Alkali- und Erdalkalihydride bevorzugt in inerten Lösungsmitteln wie Dimethylformamid, Dimethylacetamid, Methylenchlorid, Ethern, Tetrahydofuran und Toluol eingesetzt werden. Als Alkylierungsmittel kommen Alkylhalogenide, wie Alkylchlorid, Alkylbromid, besonders Alkyljodid sowie Alkyltosylate,- Mesylate, -Triflate, - Dialkylsulfate in Betracht. Die Alkylreste der Alkylierungsmittel entsprechen den zuvor getroffenen Definitionen für $R^4$. Das Reaktionsgemisch wird 0,5 bis 4 Tage, bevorzugt 1 bis 2 Tage bei Raumtemperatur gerührt und zur Trockne eingeengt. Die Aufarbeitung kann zum einen dadurch erfolgen, daß der Rückstand mit Wasser und einem Lösemittel, beispielsweise einem halogenierten Lösungsmittel, wie Tetrachlorkohlenstoff, Methylenchlorid, bevorzugt Methylenchlorid verrührt wird, wobei die Verbindung (5) sich als Feststoff durch Abfiltrieren isolieren läßt. Ferner kann die Aufarbeitung, für den Fall, daß kein Feststoff anfällt, erfolgen, indem aus dem Filtrat die Phasen des Filtrats getrennt, die wässrige Lösung mit einem halogenierten Lösungsmittel, bevorzugt Methylenchlorid, extrahiert und die vereinigten organischen Phasen getrocknet und aufgearbeitet werden. Durch chromatographische Reinigung des Rückstands erhält man die Verbindung (5).

Durch Nitrosierung einer Verbindung (5) erhält man in der vierten Stufe eine Verbindung (6).

Hierzu bieten sich erfindungsgemäß 2 Verfahrensvarianten an.

Stufe 4, Variante A:

Eine Verbindung (5) wird in einem polaren Lösungsmittel gelöst oder suspendiert, wobei als polares Lösungsmittel die zuvor genannten Lösungsmittel in Frage kommen können und Dimethylacetamid oder ein Alkohol, beispielsweise Methanol, Ethanol, Propanol, Isopropanol und Butanol bevorzugt sind. Dimethylformamid ist besonders bevorzugt. Es empfiehlt sich, das gewählte Lösungsmittel als wasserfreies, gegebenenfalls absolutes Lösungsmittel einzusetzen. Die so erhaltene Mischung wird auf unter 0° Celsius, bevorzugt -5°C, abgekühlt. Zu dieser Mischung wird ein Nitrosierungsmittel, beispielsweise ein Alkylnitrit wie Methyl-, Ethyl-, n-Propyl-, Isopropyl-, n-Butyl-, sec-Butyl-, tert-Butyl-, und Pentylnitrit, jedoch bevorzugt Isoamylnitrit gegeben, wobei die Temperatur nicht über 0°C steigen soll. Bei dieser Temperatur wird über 2 bis 48, bevorzugt 4 bis 24, besonders bevorzugt 8 bis 12 Stunden gerührt. Anschließend wird gegebenenfalls bis zur Vervollständigung der Umsetzung bei Raumtemperatur weiter gerührt. Das Lösungsmittel wird im Vakuum entfernt, der Rückstand mit einem Lösungsmittel, beispielsweise Diethylether, aufgenommen, filtriert, mit Wasser gewaschen und der so erhaltene Rückstand (im Vakuum) getrocknet.

Stufe 4, Variante B:

Eine Verbindung (5) wird mit einer wässerigen Säure versetzt, wobei als Säure organische Säuren, Ameisensäure, Essigsäure, Propionsäure, bevorzugt Essigsäure und anorganische Säuren, beispielsweise Salzsäure oder verdünnte Schwefelsäure, bevorzugt Salzsäure sowie deren Mischungen verwendet werden. Die Mischung wird auf 30 bis 100, bevorzugt 50 bis 80, besonders bevorzugt 70°C erwärmt und mit einem Nitrosierungsmittel, bevorzugt Natriumnitrit, besonders bevorzugt Natriumnitrit in Wasser, versetzt und 0,5 - 2 Stunden, bevorzugt eine Stunde bei der zuvor genannten Temperatur gehalten und 0,5 - 2 Stunden, bevorzugt eine Stunde bei 0 bis 20, bevorzugt 5 bis 15, besonders bevorzugt 10°C gehalten. Das Produkt wird filtriert, der Rückstand gewaschen und im Vakuum getrocknet.

In der fünften Stufe erfolgt die Reduktion der Nitrosoverbindung (6) zu einem Diamin-Derivat (7). Folgende Verfahrensvarianten sind hierzu u.a. anwendbar.

Stufe 5, Variante A:

Die Nitrosoverbindung (6) wird unter Rühren im Wasser suspendiert und mit einem geeigneten Reduktionsmittel versetzt. Geeignete Reduktionsmittel sind Dithionit in saurer oder alkalischer Lösung. Auch Ammoniumsulfit, Lithium-, Natrium-, Kaliumhydrogensulfit, Triethylphosphit, Triphenylphosphin oder Lithiumaluminiumhydrid können verwendet werden. Weiterhin kann die Nitrosogruppe mit Hilfe von Übergangsmetallkatalysatoren auf Basis von Palladium, Platin, Nickel oder Rhodium katalytisch mit Wasserstoffgas oder durch Transferhydrierung, beispielsweise mit Cyclohexen oder Ammoniumformiat als Wasserstoffquelle hydriert werden. Bevorzugt ist jedoch der Einsatz von Natriumdithionit. Als Basen können neben der bevorzugt eingesetzten wässrigen Ammoniaklösung gleichfalls Alkali- oder Erdalkalihydroxidlösungen, beispielsweise Lithium-, Natrium-, Kaliumhydoxid oder Magnesium-, Calzium-, Bariumhydroxidlösungen, bevorzugt jedoch die verdünnten Lösungen eingesetzt werden. Weiterhin können die Methyl-, Ethyl-, Isopropyl-, n-Propyl-, iso-Butyl-, tert-Butyl-, n-Butyl-Alkoholate der zuvor genannten Alkali- und Erdalkalimetalle verwendet werden. Diese Suspension wird auf 20 bis 100, bevorzugt 50 bis 90, besonders bevorzugt 60 bis 80°C erhitzt und mit einer Säure versetzt, bevorzugt zu Beginn, besonders bevorzugt innerhalb der ersten 30 Minuten. Mögliche Säuren sind anorganische Säuren, beispielsweise Salzsäure und Schwefelsäure sowie organische Säuren beispielsweise Ameisensäure und Essigsäure sowie deren Mischungen, wobei Schwefelsäure bevorzugt und eine 50%ige Schwefelsäure besonders bevorzugt ist. Die so erhaltene Mischung wird bis zur vollständigen Umsetzung auf Rückflußtemperatur erhitzt. Beim Abkühlen fällt das Produkt als Semisulfat aus. Weiterhin kann das Produkt durch Neutralisieren mit einer wässrigen Base, beispielsweise Lithiumhydroxid, Natriumhydroxid, Kaliumhydroxid, bevorzugt Natronlauge, besonders bevorzugt 30 Gew.-%ige Natronlauge durch Neutralisieren ausgefällt und als freie Base abfiltriert werden.

Stufe 5, Variante B:

Zu einer Mischung aus wässriger Base und einem Alkohol wird unter Rühren die Nitrosoverbindung (6) zugegeben. Anschließend wird, nachdem die Nitrosoverbindung gelöst oder suspendiert ist, das Reduktionsmittel, bevorzugt in Lösung, zugegeben. Als Reduktionsmittel kommen die in Methode A genannten Reduktionsmittel in Frage, wobei eine wässrige Lösung von Natriumdithionit besonders bevorzugt ist. Anschließend wird 0,5 bis 6, bevorzugt 1 bis 4, besonders bevorzugt 1,5 bis 3 Stunden bei Raumtemperatur gerührt. Das ausgefallene Produkt wird durch

Abfiltrieren isoliert.

In der sechsten Stufe werden aus den Diaminen (7) die isomeren Verbindungen (8) hergestellt.

Hierzu wird die Aminoverbindung (7) in einem organischen Lösungsmittel suspendiert und wird mit einer organischen Base versetzt. Nachdem die Mischung 40 bis 60 Minuten, bevorzugt 20 bis 50 Minuten, besonders bevorzugt 25 bis 45 Minuten bei Raumtemperatur gehalten wurde, wird die Mischung auf 0 bis 15°C, bevorzugt 5 bis 10°C abgekühlt und mit einem Carbonsäurederivat, worin der Rest $R^2$ wie zuvor definiert ist, versetzt. Geeignete organische Lösungsmittel sind Dimethylformamid, Dimethylacetamid, Methylenchlorid, Toluol und Tetrahydrofuran, wobei Dimethylformamid bevorzugt und wasserfreies, gegebenenfalls absolutes Dimethylformamid besonders bevorzugt ist. Geeignete organische Basen sind Dimethylaminopyridin, Pyridin, tert. Amine, beispielsweise Trimethylamin, Triethylamin, Diisopropylethylamin, DBU (Diazabicycloundecen) oder eine der zuvor genannten anorganischen Basen, insbesondere Alkali- oder Erdalkalicarbonate oder -hydrogencarbonate. Besonders geeignet als Base ist jedoch Dimethylaminopyridin. Erfindungsgemäße Carbonsäurederivate sind Carbonsäurehalogenide, bevorzugt Carbonsäurechloride oder auch Carbonsäuren, wenn sie in geeigneter Weise aktiviert werden. Dieses kann beispielsweise durch Umsetzung mit Chlorameisensäureestern, Carbonyldiimidazol, Carbodiimiden, beispielsweise Dicyclohexylcarbodiimid, EDAC, oder Benzotriazolen, beispielsweise HOBt (1-Hydroxy-1-H-benzotriazol) und TBTU erfolgen. Gleichfalls kann an Stelle des Carbonsäurederivats auch der entsprechende Aldehyd eingesetzt werden und das Zwischenprodukt mit Eisen-(II)-Chlorid, Azodicarbonsäureestern und anderen Oxidationsmitteln oxidiert werden. Anschließend wird die Mischung über 0,5 bis 4 Stunden, bevorzugt 1 bis 3 Stunden, besonders bevorzugt 2 Stunden, bei 5 bis 20, bevorzugt 7 bis 15, besonders bevorzugt 10°C und über Nacht bei Raumtemperatur gerührt und das Lösungsmittel anschließend im Vakuum entfernt. Der Rückstand wird in Wasser aufgenommen, der Feststoff abfiltriert, gewaschen und gegebenenfalls durch Umkristallisation oder Chromatographie, bevorzugt Säulenchromatographie, gereinigt.

In der siebten Stufe erfolgt die Ringschlußreaktion einer Verbindung (8) zur Verbindung (9) gemäß Methode A .

Stufe 7, Methode A :

Eine Verbindung (8) wird in einem Alkohol suspendiert und mit einer Base 24 Stunden unter Rückfluß erhitzt. Nach dem Abkühlen wird der Ansatz sauer gestellt, der Feststoff abfiltriert und getrocknet. Gegebenenfalls wird das Produkt durch Umkristallisation oder Chromatographie, bevorzugt Säulenchromatographie, gereinigt. Als Base kommen hier die Alkali- und Erdalkalihydroxide, beispielsweise Hydroxide des Lithiums, Natriums, Kaliums, Calziums und Bariums sowie deren Mischungen als wäßrige Lösung oder gegebenenfalls in Gemischen mit einem Alkohol und/oder mit einem wassermischbaren Ether versetzt, in Frage. Geeignete Alkohole sind Methanol, Ethanol, n-Propanol, Isopropanol, n-Butanol und tert.-Butanol. Als Ether eignen sich insbesondere cyclische Ether, beispielsweise Tetrahydrofuran, Dioxan.

Nach Analogieverfahren kann der Ringschluß auch mit anorganischen Säurechloriden wie Thionylchlorid, Phosphoroxychlorid oder Phosphorpentachlorid sowie mit Polyphosphorsäure durchgeführt werden.

Ein weiteres Analogieverfahren besteht darin, das Carbonsäureamid unter sauren Bedingungen mit Mineralsäure/Methanol, bevorzugt Salzsäure/Methanol oder basischen Bedingungen, Alkalimetall/Methanol, bevorzugt Natrium/Methanol umzusetzen und anschließend thermisch zu cyclisieren.

Die vorliegende Erfindung wird anhand beispielhafter Synthesevorschriften näher erläutert. Die Beispiele dienen der Illustration, der Erfindung.

**Stufe 1: Synthese von 5-substituierten 3-Amino-1,2,4-triazolen (3):**

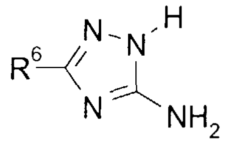

(3)

Das unsubstituierte 3-Amino-1,2,4-triazol ist käuflich, die benötigten 5-substituierten 3-Amino-1,2,4-triazole können entsprechend literaturbekannten Verfahren hergestellt werden, die beispielsweise in J. Chem. Soc. 1929, 816, J. Org. Chem. 1926, 1729 oder Org. Synthesis 26, 11 veröffentlicht sind. Die Carbonsäuren $R^6$-COOH bzw. Nitrile $R^6$-CN sind käuflich erhältlich oder können nach literaturbekannten Verfahren hergestellt werden.

Tabelle 1:

| Nr. | $R^6$ | Ausbeute (%) | Fp (°C) |
|---|---|---|---|
| 1.1. | -H | käuflich | - |
| 1.2. | -Methyl | 76 | 145-148 |
| 1.3. | -Ethyl | 75 | 152 |
| 1.4. | -n-Propyl | 62 | 140-143 |
| 1.5. | -i-Propyl | 67 | 103-104 |
| 1.6. | -n-Butyl | 62 | 118 |
| 1.7. | -t-Butyl | 39 | 130-131 |
| 1.8. | Benzyl- | 70 | 167-169 |
| 1.9. | Cyclopentyl- | 64 | 168-172 |
| 1.10. | 2-Furyl- (Semisulfat) | 76 | 207-208 |
| 1.11. | Phenyl- | 68 | 185-186 |
| 1.12. | Cyclohexylmethyl- | 51 | 186-188 |
| 1.13. | 2-Phenylethyl- | 53 | 139-140 |
| 1.14. | (4-MeO-Ph)-O-$CH_2$- | 41 | - |
| 1.15. | (4-MeO-Ph)-$CH_2$-O-$CH_2$- | 54 | 153 |
| 1.16. | (4-Ph-$CH_2$O-Ph)-$CH_2$- | 57 | 196-200 |
| 1.17. | 4-Fluorbenzyl- | 100 | 163-164 |
| 1.18. | 3,4-Difluorbenzyl- | 81 | 135-137 |
| 1.19. | 3-Pyridylmethyl- | 58 | 192-196 |

**Stufe 2: Synthese der Verbindungen (4)**

Allgemeine Arbeitsvorschrift:

2,3 g (0,1 Mol) Natrium werden in 10 ml abs. Ethanol gelöst, dann gibt man 14,7 g (0,13 Mol) Cyanessigsäureethylester zu. Die Mischung wird 1 - 2 h bei Raumtemperatur gerührt, anschließend mit 0,1 Mol des gewünschten, 5-substituierten 3-Aminotriazols versetzt und 4 - 6 h unter Rückfluß erhitzt. Das abgekühlte Reaktionsgemisch wird mit Wasser versetzt und unter Rühren sauer gestellt, dann wird der Feststoff abfiltriert, gewaschen und getrocknet.

Nach diesem Verfahren wurden u.a. die folgenden Verbindungen hergestellt:

Tabelle 2:

| Nr | $R^6$ | Ausbeute (%) | Fp (°C) |
|---|---|---|---|
| 2.1. | -H | 73 | >370 |
| 2.2. | -Methyl | 76 | >300 |
| 2.3. | -Ethyl | 81 | >300 |
| 2.4. | -n-Propyl | 80 | 290 (Zers.) |
| 2.5. | -i-Propyl | 81 | 294 (Zers.) |
| 2.6. | -n-Butyl | 77 | 256 |
| 2.7. | -t-Butyl | 78 | >300 |
| 2.8. | Benzyl- | 82 | 300 (Zers.) |
| 2.9. | Cyclopentyl- | 83 | 310 (Zers.) |

Tabelle 2: (fortgesetzt)

| Nr | $R^6$ | Ausbeute (%) | Fp (°C) |
|---|---|---|---|
| 2.10. | 2-Furyl- | 61 | - |
| 2.11. | Phenyl- | 75 | - |
| 2.12. | Cyclohexylmethyl- | 81 | > 300 |
| 2.13. | 2-Phenylethyl- | 83 | 295-296 |
| 2.14. | (4-MeO-Ph)-O-$CH_2$- | 68 | 302 |
| 2.15. | (4-MeO-Ph)-$CH_2$-O-$CH_2$- | 89 | 245 |
| 2.16. | (4-$PhCH_2O$-Ph)-$CH_2$- | 96 | 296-298 |
| 2.17. | 4-Fluorbenzyl- | 79 | 300-302 |
| 2.18. | 3,4-Difluorbenzyl- | 62 | 290-292 |
| 2.19. | 3-Pyridylmethyl- | 82 | >300 |

**Stufe 3: Synthese der alkylierten Triazolopyrimidin-Derivate (5):**

Allgemeine Arbeitsvorschrift:

66 mMol des betreffenden Triazolopyrimidins (4) werden in der 10 - 20fachen Menge wasserfreiem Dimethylformamid gelöst und mit 76 mMol Kaliumcarbonat und 76 mMol des gewünschten Alkyljodids versetzt. Das Reaktionsgemisch wird 1 - 2 Tage bei Raumtemperatur gerührt und zur Trockne eingeengt. Der Rückstand wird mit Wasser und Methylenchlorid verrührt. In vielen Fällen läßt sich nach dieser Behandlung (5) als Feststoff abfiltrieren und dadurch isolieren. Falls beim Verrühren kein Feststoff anfällt werden die Phasen des Filtrats getrennt, die wäßrige Phase wird mit Methylenchlorid ausgeschüttelt, und die vereinigten organischen Phasen werden über Magnesiumsulfat getrocknet und zur Trockne eingeengt. Durch chromatographische Reinigung des Rückstand wird (5) isoliert.
Die benötigten Alkylhalogenide R'-X (mit $R'=R^4$) sind käuflich erhältlich oder können nach literaturbekannten Verfahren hergestellt werden.

Nach diesem Verfahren wurden u.a.die folgenden Substanzen hergestellt:

Tabelle 3:

| Nr. | $R^6$ | $R^4$ | Ausbeute (%) | Fp (°C) |
|---|---|---|---|---|
| 3.1 | -H | -Methyl | 80 | 310(Zers.) |
| 3.2 | -H | -n-Propyl | 58 | 184 - 186 |
| 3.3 | -Methyl | -n-Propyl | 61 | 228 |
| 3.4 | -Ethyl | -n-Propyl | 65 | 194 |
| 3.5 | -n-Propyl | -n-Propyl | 69 | 170 |
| 3.6 | -i-Propyl | -n-Propyl | 50 | 138 |
| 3.7 | -Butyl | -n-Propyl | 62 | 167 |
| 3.8 | -t-Butyl | -n-Propyl | 64 | 164 - 165 |
| 3.9 | 2-Furyl- | -n-Propyl | 45 | 260 - 262 |
| 3.10 | Cyclopentyl- | -n-Propyl | 64 | 148 - 150 |
| 3.11 | Benzyl- | -n-Propyl | 60 | 208 |
| 3.12 | Benzyl- | -Methyl | 52 | 296 |
| 3.13 | Benzyl- | -Ethyl | 58 | 213 |
| 3.14 | Benzyl | -i-Propyl | 29 | 232 |
| 3.15 | Benzyl- | -n-Butyl | 55 | 175 |

Tabelle 3: (fortgesetzt)

| Nr. | $R^6$ | $R^4$ | Ausbeute (%) | Fp (°C) |
|---|---|---|---|---|
| 3.16 | Benzyl- | -Pentyl | 63 | 188 |
| 3.17 | Benzyl- | Benzyl- | 51 | 180 - 181 |
| 3.18 | Phenyl- | -n-Propyl | 30 | 270 - 271 |
| 3.19 | Cyclohexylmethyl- | -Ethyl | 67 | 210-212 |
| 3.20 | 2-Phenylethyl- | -Ethyl | 29 | 173-174 |
| 3.21 | (4-MeO-Ph)-O-$CH_2$- | -Ethyl | 58 | 177-180 |
| 3.22 | (4-MeO-Ph)-$CH_2$-O-$CH_2$- | -Ethyl | 100 | -* |
| 3.23 | (4-Ph$CH_2$-O-Ph)-$CH_2$- | -Ethyl | 63 | 240-242 |
| 3.24 | 4-Fluorbenzyl- | -Ethyl | 53 | - |
| 3.25 | 3,4-Difluorbenzyl- | -Ethyl | 61 | 228 |
| 3.26 | 3-Pyridylmethyl- | -Ethyl | 94 | 186-188 |

*Isomerentrennung auf Folgestufe

**Stufe 4: Synthese der Nitrosoverbindungen (6)**

Allgemeine Arbeitsvorschriften (Variante A):

10 mmol der Verbindungen (5) werden in 10-30 ml wasserfreiem Dimethylformamid gelöst oder suspendiert, und die Mischung wird auf -5 °C abgekühlt. Man gibt 20 mMol Isoamylnitrit so zu, daß die Temperatur nicht über 0 °C steigt und rührt bei dieser Temperatur 4 - 24 h, anschließend bei Bedarf noch bis zur Vervollständigung der Reaktion bei Raumtemperatur. Das Lösungsmittel wird im Vakuum entfernt, der Rückstand mit Ether verrührt, abfiltriert, mit Wasser gewaschen und im Vakuum getrocknet.

Nach diesem Verfahren wurden u.a. die folgenden Substanzen hergestellt:

Tabelle 4a:

| Nr. | $R^6$ | $R^4$ | Ausbeute (%) | Fp (°C) |
|---|---|---|---|---|
| 4.1 | -H | -Methyl | 79 | 248 |
| 4.2 | -H | -n-Propyl | 81 | 211 (Zers.) |
| 4.3 | -Methyl | -n-Propyl | 88 | 226 (Zers.) |
| 4.4 | -Ethyl | -n-Propyl | 94 | 190 |
| 4.5 | -n-Propyl | -n-Propyl | 96 | 206 (Zers.) |
| 4.6 | -i-Propyl | -n-Propyl | 72 | 210 (Zers.) |
| 4.7 | -n-Butyl | -n-Propyl | 95 | 196 (Zers.) |
| 4.8 | -t-Butyl | -n-Propyl | 93 | 200 (Zers.) |
| 4.9 | 2-Furyl- | -n-Propyl | 85 | 261 |
| 4.10 | Cyclopentyl- | -n-Propyl | 97 | 224 (Zers.) |
| 4.11 | Benzyl- | -n-Propyl | 95 | 208 (Zers.) |
| 4.12 | Benzyl- | -Methyl | 96 | 233 (Zers.) |
| 4.13 | Benzyl- | -Ethyl | 97 | 226 (Zers.) |
| 4.14 | Benzyl | -i-Propyl | 88 | 190 (Zers.) |
| 4.15 | Benzyl- | -n-Butyl | 93 | 196 |
| 4.16 | Benzyl- | -n-Pentyl | 74 | 190 (Zers.) |

Tabelle 4a: (fortgesetzt)

| Nr. | $R^6$ | $R^4$ | Ausbeute (%) | Fp (°C) |
|---|---|---|---|---|
| 4.17 | Benzyl- | Benzyl- | 96 | 236 |
| 4.18 | Phenyl- | -n-Propyl | 92 | 257 - 258 (Zers.) |
| 4.19 | Cyclohexylmethyl- | -Ethyl | 83 | 208-209 |
| 4.20 | 2-Phenylethyl- | -Ethyl | 90 | 200-201 |
| 4.21 | (4-MeO-Ph)-O-$CH_2$- | -Ethyl | 93 | 214-216 |
| 4.22 | (4-MeO-Ph)-$CH_2$-O-$CH_2$- | -Ethyl | 56 | 156-158 |
| 4.23 | (4-$PhCH_2$-O-Ph)-$CH_2$- | -Ethyl | 97 | 201 Zers. |
| 4.24 | 4-Fluorbenzyl- | -Ethyl | 89 | 241-242 |
| 4.25 | 3,4-Difluorbenzyl- | -Ethyl | 97 | 234-236 |
| 4.26 | 3-Pyridylmethyl- | -Ethyl | 44 | 229 |

**Stufe 5: Synthese der Diamine (7):**

Allgemeine Arbeitsvorschrift (Variante A):

12,7 mMol der Nitrosoverbindung (6) werden unter Rühren in 86 ml $H_2O$ suspendiert und mit 4,9 g (2,9 mMol) $Na_2S_2O_4$ versetzt. Die Suspension wird auf 80°C erhitzt, und innerhalb von 30 Min. werden 15 ml 50%ige $H_2SO_4$ zugegeben. Die Mischung wird bis zur vollständigen Umsetzung unter Rückfluß gekocht. Nach dem Abkühlen fällt in den meisten Fällen das Produkt als Semisulfat aus. Sollte kein Feststoff ausfallen, neutralisiert man mit 30%iger Natronlauge und filtriert die ausgefallene freie Base ab.

Allgemeine Arbeitsvorschrift (Variante B):

Zu einer Mischung aus 175 ml 25%igem wäßrigem Ammoniak und 36 ml Ethanol gibt man unter Rühren 11,5 mMol Nitrosoverbindung (6). Anschließend rührt man bei 30°C, bis sich die Nitrosoverbindung weitgehend gelöst hat, tropft eine Lösung von 6,1 g (34,7 mMol) $Na_2S_2O_4$ in 57 ml $H_2O$ zu und rührt weitere 1,5 - 3 h bei Raumtemperatur. Sollte anschließend noch Ausgangsverbindung vorhanden sein, gibt man 10% der o.a. Menge an $Na_2S_2O_4$ nach und rührt bis zur Beendigung der Umsetzung. Das Produkt wird durch Abfiltrieren isoliert.
Nach einer dieser Varianten (A oder B, s. Tabelle 5) wurden u.a. die in Tabelle 5 beschriebenen Verbindungen hergestellt:

Tabelle 5:

| Nr. | $R^6$ | $R^4$ | Variante | Ausbeute in % | Salzform | Schmelzpunkt in °C |
|---|---|---|---|---|---|---|
| 5.1 | -H | -Methyl | A | 65 | - | 267 (Zers.) |
| 5.2 | -H | -n-Propyl | A | 65 | - | 185-188 |
| 5.3 | -Methyl | -n-Propyl | A | 70 | - | 174 |
| 5.4 | -Ethyl | -n-Propyl | A | 78 | - | 170-172 |
| 5.5 | -n-Propyl | -n-Propyl | A | 82 | - | 148-150 |
| 5.6 | -i-Propyl | -n-Propyl | A | 52 | - | 193 |
| 5.7 | -n-Butyl | -n-Propyl | A | 71 | - | 140-142 |
| 5.8 | -t-Butyl | -n-Propyl | B | 90 | - | 168 |
| 5.9 | Cyclopentyl- | -n-Propyl | A | 48 | Semisulfat | 195-197 |
| 5.10 | 2-Furyl- | -n-Propyl | B | 75 | - | 204-205 |
| 5.11 | Benzyl | -i-Propyl | A | 8 | Semisulfat | 223 |
| 5.12 | Benzyl- | -n-Propyl | A | 62 | Semisulfat | 217 |
| 5.13 | Benzyl- | -Methyl | B | 91 | - | 248 |

| Nr. | $R^6$ | $R^4$ | Variante | Ausbeute in % | Salzform | Schmelzpunkt in $^oC$ |
|---|---|---|---|---|---|---|
| 5.14 | Benzyl- | -Ethyl | B | 95 | - | 175 |
| 5.15 | Benzyl- | -n-Butyl | A | 48 | Semisulfat | 203-205 |
| 5.16 | Benzyl- | -n-Pentyl | B | 92 | - | 146 |
| 5.17 | Benzyl- | Benzyl- | B | 80 | - | 273 |
| 5.18 | Phenyl- | -n-Propyl | A | 79 | Semisulfat | 260 |
| 5.19 | Cyclohexylmethyl- | -Ethyl | B | 94 | - | 158-160 |
| 5.20 | 2-Phenylethyl- | -Ethyl | B | 94 | - | 204-205 |
| 5.21 | (4-MeO-Ph)-O-$CH_2$- | -Ethyl | B | 86 | - | 154-156 |
| 5.22 | (4-MeO-Ph)-$CH_2$-O-$CH_2$- | -Ethyl | B | 88 | - | 133-139 |
| 5.23 | (4-Ph$CH_2$-O-Ph)-$CH_2$- | -Ethyl | B | 84 | - | 155 |
| 5.24 | 4-Fluorbenzyl- | -Ethyl | B | 88 | - | 202-205 |
| 5.25 | 3,4-Difluorbenzyl- | -Ethyl | B | 95 | - | 209-212 |
| 5.26 | 3-Pyridylmethyl- | -Ethyl | B | 82 | - | 179-180 |

**Stufe 6: Synthese der Amide (8):**

Allgemeine Arbeitsvorschrift (Variante A):

30 mMol Diaminoverbindung (7) werden in 165 ml wasserfreiem Dimethylformamid suspendiert und mit 45 mMol (im Falle von Semisulfaten mit 78 mMol) 4-Dimethylaminopyridin versetzt. Man rührt 30 Min. bei Raumtemperatur, kühlt die Mischung auf 5- 10°C und gibt eine Lösung aus 39 mMol des gewünschten Carbonsäurechlorids in 16 ml wasserfreiem Dimethylformamid zu. Anschließend wird 2 h bei 10°C und über Nacht bei Raumtemperatur gerührt, dann wird das Lösungsmittel im Vakuum entfernt. Der Rückstand wird auf Wasser gegeben, der Feststoff abfiltriert, gewaschen und ggfs. durch Umkristallisation oder Chromatographie gereinigt.

Die benötigten Carbonsäurechloride sind käuflich oder können nach literaturbekannten Verfahren hergestellt werden.

Nach diesem Verfahren wurden u.a. die in Tabelle 6a beschriebenen Verbindungen (8) hergestellt:

Tabelle 6a:

| Nr. | $R^6$ | $R^4$ | $R^2$ | Ausbeute in % | Schmelzpunkt in °C |
|---|---|---|---|---|---|
| 6.1 | -H | -Methyl | Benzyl- | 35 | 306 |
| 6.2 | -H | -n-Propyl | Benzyl- | 65 | 178 |
| 6.3 | -H | -n-Propyl | Cyclopentyl- | 95 | 192 |
| 6.4 | -Methyl | -n-Propyl | Cyclopentyl- | 71 | 208 |
| 6.5 | -Ethyl | -n-Propyl | Cyclopentyl- | 72 | 209 |
| 6.6 | -Ethyl | -n-Propyl | -Ethyl | 77 | - |
| 6.7 | -n-Propyl | -n-Propyl | Cyclopentyl- | 90 | 210 |
| 6.8 | -n-Propyl | -n-Propyl | Phenyl- | 68 | 205 |
| 6.9 | -n-Propyl | -n-Propyl | 4-Fluorbenzyl- | 84 | 200 |
| 6.10 | -i-Propyl | -n-Propyl | Cyclopentyl- | 71 | 148 |
| 6.11 | -n-Butyl | -n-Propyl | Cyclopentyl- | 66 | 173 |
| 6.12 | -t-Butyl | -n-Propyl | Cyclopentyl- | 60 | 100 |
| 6.13 | Cyclopentyl- | -n-Propyl | -Ethyl | 76 | 205 |
| 6.14 | Cyclopentyl- | -n-Propyl | -Methyl | 70 | 243 |
| 6.15 | Cyclopentyl- | -n-Propyl | Benzyl- | 76 | 160 |
| 6.16 | 2-Furyl- | -n-Propyl | Cyclopentyl- | 74 | - |
| 6.17 | 2-Furyl- | -n-Propyl | -Ethyl | 73 | - |
| 6.18 | Benzyl- | Benzyl- | Cyclopentyl- | 90 | - |
| 6.19 | Benzyl- | -Ethyl | 3-Tetrahydrofuranyl- | 60 | 227 |
| 6.20 | Benzyl- | -Ethyl | 4-Tetrahydropyranyl- | 65 | 237 |

| Nr. | $R^6$ | $R^4$ | $R^2$ | Ausbeute in % | Schmelzpunkt in °C |
|---|---|---|---|---|---|
| 6.21 | Benzyl- | -Ethyl | 2-Pyridyl- | 63 | 223 |
| 6.22 | Benzyl- | -Ethyl | 4-Pyridyl- | 55 | |
| 6.23 | Benzyl- | -n-Propyl | -Ethyl | 61 | 183 |
| 6.24 | Benzyl- | -n-Propyl | -n-Propyl | 62 | 166 |
| 6.25 | Benzyl- | -Methyl | Cyclopentyl- | 88 | 205-208 |
| 6.26 | Benzyl- | -Ethyl | Cyclopentyl- | 90 | 188-189 |
| 6.27 | Benzyl- | -n-Propyl | Cyclopentyl- | 77 | 170 |
| 6.28 | Benzyl- | -n-Butyl | Cyclopentyl- | 60 | 163 |
| 6.29 | Benzyl- | -n-Pentyl | Cyclopentyl- | 67 | 162 |
| 6.30 | Phenyl- | -n-Propyl | -Ethyl | 47 | 165-166 |
| 6.31 | Phenyl- | -n-Propyl | Cyclopentyl- | 68 | >300 |
| 6.32 | Benzyl- | -Ethyl | 2-Pyridyl- | 40 | 223 |
| 6.33 | Benzyl- | -Ethyl | 4-Pyridyl- | 55 | - |
| 6.34 | Benzyl- | -Ethyl | 3-Pyridyl- | 88 | 206 |
| 6.35 | Benzyl- | -Ethyl | Cyclohexyl- | 86 | 186-187 |
| 6.36 | 3-Pyridylmethyl- | -Ethyl | Cyclopentyl- | 73 | 163-165 |
| 6.37 | 3-Pyridylmethyl- | -Ethyl | -Ethyl | 71 | 186-187 |
| 6.38 | 2-Furyl- | -n-Propyl | 2-Furyl- | 81 | 228 |

| Nr. | $R^6$ | $R^4$ | $R^2$ | Ausbeute in % | Schmelzpunkt in $^oC$ |
|---|---|---|---|---|---|
| 6.39 | Cyclohexylmethyl- | -Ethyl | -Ethyl | 81 | 204-207 |
| 6.40 | Cyclohexylmethyl- | -Ethyl | Cyclopentyl- | 70 | 196-198 |
| 6.41 | 2-Phenylethyl- | -Ethyl | -Ethyl | 95 | 182-183 |
| 6.42 | 2-Phenylethyl- | -Ethyl | Cyclopentyl- | 81 | 155-156 |
| 6.43 | Cyclopentyl- | -n-Propyl | 3-Tetrahydrofuranyl- | 65 | 179-180 |
| 6.44 | Cyclopentyl- | -n-Propyl | Cyclopentyl- | 41 | 306-308 |
| 6.45 | (4-MeO-Ph)- $OCH_2$- | -Ethyl | -Ethyl | 93 | 160-164 |
| 6.46 | (4-MeO-Ph)- $OCH_2$- | -Ethyl | Cyclopentyl- | 76 | 176-178 |
| 6.47 | (4-MeO-Ph)-$CH_2$-O-$CH_2$- | -Ethyl | Cyclopentyl- | 91 | 133-136 |
| 6.48 | (4-$PhCH_2O$-Ph)-$CH_2$- | -Ethyl | Cyclopentyl- | 86 | 190 |
| 6.49 | 4-Fluorbenzyl- | -Ethyl | Cyclopentyl- | 62 | 206-208 |
| 6.50 | 4-Fluorbenzyl- | -Ethyl | tert.-Butyl- | 53 | - |
| 6.51 | 4-Fluorbenzyl- | -Ethyl | 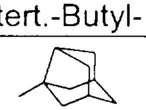 | 91 | - |
| 6.52 | 3,4-Difluorbenzyl- | -Ethyl | Cyclopentyl- | 100 | 222-224 |
| 6.53 | 3,4-Difluorbenzyl- | -Ethyl | 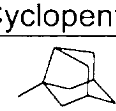 | 100 | - |
| 6.54 | Benzyl- | -Ethyl | 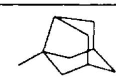 | 87 | - |
| 6.55 | 3-Pyridylmethyl- | -Ethyl | 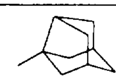 | 79 | 134-137 |

**Stufe 7: Ringschlußreaktionen zu den Verbindungen (9)**

Allgemeine Arbeitsvorschrift (Methode A, Variante A):

14,4 mMol (8) werden in 75,5 ml $H_2O$ und 37,8 ml Ethanol suspendiert und mit 17,4 ml 50%iger NaOH und 4,82 g (65 mMol) $Ca(OH)_2$ 24 h unter Rückfluß gekocht. Man läßt den Ansatz abkühlen, stellt ihn sauer, filtriert den Feststoff ab und trocknet ihn. Das Produkt wird gegebenenfalls durch Umkristallisation oder Chromatographie gereinigt. Nach diesem Verfahren wurden u.a. die in Tabelle 7a beschriebenen Verbindungen (9) hergestellt.

Tabelle 7a:

| Nr. | $R^6$ | $R^4$ | $R^2$ | Ausbeute in % | Schmelzpunkt in °C |
|---|---|---|---|---|---|
| 7.1 | -H | -Methyl | Benzyl- | 35 | 306 |
| 7.2 | -H | -n-Propyl | Benzyl- | 68 | 222-223 |
| 7.3 | -H | -n-Propyl | Cyclopentyl- | 59 | 250 |
| 7.4 | -Methyl | -n-Propyl | Cyclopentyl- | 62 | 262-264 |
| 7.5 | -Ethyl | -n-Propyl | Cyclopentyl- | 58 | 253 |
| 7.6 | -Ethyl | -n-Propyl | -Ethyl | 68 | 229 |
| 7.7 | -n-Propyl | -n-Propyl | Cyclopentyl- | 75 | 256 |
| 7.8 | -n-Propyl | -n-Propyl | Phenyl- | 49 | 300 (Zers.) |
| 7.9 | -n-Propyl | -n-Propyl | 4-Fluorbenzyl- | 65 | 221 |
| 7.10 | -i-Propyl | -n-Propyl | Cyclopentyl- | 62 | 260-261 |
| 7.11 | -n-Butyl | -n-Propyl | Cyclopentyl- | 72 | 246 |
| 7.12 | -t-Butyl | -n-Propyl | Cyclopentyl- | 64 | 310 |
| 7.13 | Cyclopentyl- | -n-Propyl | -Ethyl | 85 | 245-248 |
| 7.14 | Cyclopentyl- | -n-Propyl | -Methyl | 56 | 248-250 |
| 7.15 | Cyclopentyl- | -n-Propyl | Benzyl- | 70 | 233 |
| 7.16 | 2-Furyl- | -n-Propyl | Cyclopentyl- | 61 | >300 (Zers.) |
| 7.17 | 2-Furyl- | -n-Propyl | -Ethyl | 44 | >300 (Zers.) |
| 7.18 | Benzyl- | Benzyl- | Cyclopentyl- | 13 | 270 |
| 7.19 | Benzyl- | -Ethyl | 3-Tetrahydrofuranyl- | 60 | 227 |

| Nr. | $R^6$ | $R^4$ | $R^2$ | Ausbeute in % | Schmelzpunkt in °C |
|---|---|---|---|---|---|
| 7.20 | Benzyl- | -Ethyl | 4-Tetrahydropyranyl- | 64 | 237 |
| 7.21 | Benzyl- | -Ethyl | 2-Pyridyl- | 21 | 287 |
| 7.22 | Benzyl- | -Ethyl | 4-Pyridyl- | 8 | 346 |
| 7.23 | Benzyl- | -n-Propyl | -Ethyl | 57 | 223 |
| 7.24 | Benzyl- | -n-Propyl | -n-Propyl | 53 | 202 |
| 7.25 | Benzyl- | -Methyl | Cyclopentyl- | 13 | 265-267 |
| 7.26 | Benzyl- | -Ethyl | Cyclopentyl- | 56 | 242 |
| 7.27 | Benzyl- | -n-Propyl | Cyclopentyl- | 67 | 234-235 |
| 7.28 | Benzyl- | -n-Butyl | Cyclopentyl- | 53 | 238-240 |
| 7.29 | Benzyl- | -n-Pentyl | Cyclopentyl- | 56 | 231-236 |
| 7.30 | Phenyl- | -n-Propyl | -Ethyl | 97 | >300 (Zers.) |
| 7.31 | Phenyl- | -n-Propyl | Cyclopentyl- | 78 | >300 (Zers.) |
| 7.32 | Benzyl- | -Ethyl | 3-Pyridyl- | 11 | 331-332 |
| 7.33 | Benzyl- | -Ethyl | Cyclohexyl- | 74 | 260-262 |
| 7.34 | 3-Pyridylmethyl- | -Ethyl | Cyclopentyl- | 60 | 261-263 |
| 7.35 | 3-Pyridylmethyl- | -Ethyl | -Ethyl_ | 62 | 225-227 |
| 7.36 | 2-Furyl- | -n-Propyl | 2-Furyl- | 9 | 385-387 |
| 7.37 | Cyclohexylmethyl- | -Ethyl | -Ethyl | 73 | 230-232 |
| 7.38 | Cyclohexylmethyl- | -Ethyl | Cyclopentyl- | 62 | 273-274 |

| Nr. | $R^6$ | $R^4$ | $R^2$ | Ausbeute in % | Schmelzpunkt in °C |
|---|---|---|---|---|---|
| 7.39 | 2-Phenylethyl- | -Ethyl | -Ethyl | 31 | 282-283 |
| 7.40 | 2-Phenylethyl- | -Ethyl | Cyclopentyl- | 53 | 292-294 |
| 7.41 | Cyclopentyl- | n-Propyl | 3-Tetrahydrofuranyl- | 56 | 289-290 |
| 7.42 | Cyclopentyl- | n-Propyl | Cyclopentyl- | 41 | 306-308 |
| 7.43 | (4-MeO-Ph)-$OCH_2$- | -Ethyl | -Ethyl | 47 | 225 |
| 7.44 | (4-MeO-Ph)-$OCH_2$- | -Ethyl | Cyclopentyl- | 20 | 234-236 |
| 7.45 | (4-MeO-Ph)-$CH_2$-O-$CH_2$- | -Ethyl | Cyclopentyl- | 70 | 182 |
| 7.46 | (4-$PhCH_2O$-Ph)-$CH_2$- | -Ethyl | Cyclopentyl- | 70 | 251 |
| 7.47 | 4-Fluorbenzyl- | -Ethyl | Cyclopentyl- | 71 | 252-254 |
| 7.48 | 4-Fluorbenzyl- | -Ethyl | -tert.-Butyl | 73 | 262-264 |
| 7.49 | 4-Fluorbenzyl- | -Ethyl | 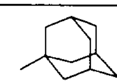 | 53 | 337-339 |
| 7.50 | 4-Fluorbenzyl- | -Ethyl | 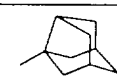 | 45 | 325-327 |
| 7.51 | 3,4-Difluorbenzyl- | -Ethyl | Cyclopentyl- | 60 | 264-266 |
| 7.52 | 3,4-Difluorbenzyl- | -Ethyl | 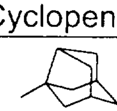 | 58 | 315-317 |
| 7.53 | Benzyl- | -Ethyl | 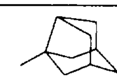 | 67 | 320-322 |
| 7.54 | 3-Pyridylmethyl- | -Ethyl | 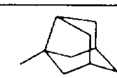 | 57 | 330-332 |

Tabelle 8 faßt die in Analogie zu den zuvor beschriebenen Verfahren hergestellten Verbindungen zusammen:

Tabelle 8:

| Beispiel Nr. | $-R^2$ | $-R^4$ | $-R^6$ | Schmelzpunkt in °C |
|---|---|---|---|---|
| 1 | -Ethyl | -n-Propyl | 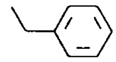 | 223 |
| 2 | -n-Propyl | -n-Propyl | 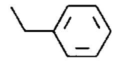 | 202 |
| 3 | 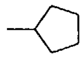 | -Methyl | 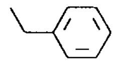 | 265-267 |
| 4 | 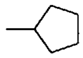 | -Ethyl | 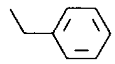 | 242 |
| 5 | 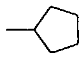 | -n-Propyl | 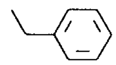 | 234-235 |
| 6 | 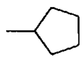 | -n-Butyl | 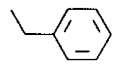 | 238-240 |
| 7 | 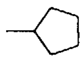 | 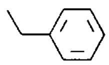 | 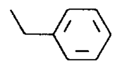 | 270 |
| 8 | 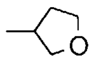 | -Ethyl | 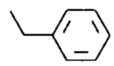 | 237 |
| 9 | 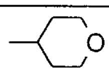 | -Ethyl | 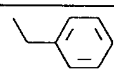 | 227 |
| 10 | $-CH_3$ | -n-Propyl | 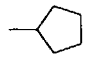 | 248-250 |
| 11 | -Ethyl | -n-Propyl | 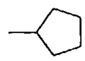 | 245-248 |
| 12 | 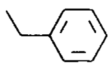 | -n-Propyl | 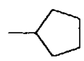 | 233 |
| 13 | 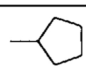 | -n-Pentyl | 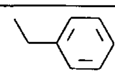 | 231-236 |
| 14 | 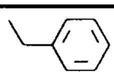 | $-CH_3$ | -H | 306 |
| 15 | 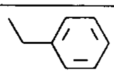 | -n-Propyl | -H | 222-223 |
| 16 | 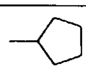 | -Methyl | -H | 285 |

| Beispiel Nr. | $-R^2$ | $-R^4$ | $-R^6$ | Schmelzpunkt in °C |
|---|---|---|---|---|
| 17 | 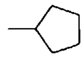 | -Ethyl | -H | 249-253 |
| 18 | 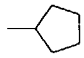 | -n-Propyl | -H | 250 |
| 19 | 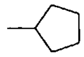 | -n-Propyl | $-CH_3$ | 262-264 |
| 20 | 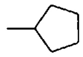 | -n-Propyl | -Ethyl | 253 |
| 21 | 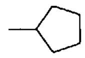 | -n-Propyl | -n-Propyl | 256 |
| 22 | 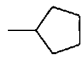 | -n-Propyl | -i-Propyl | 260-261 |
| 23 | 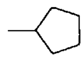 | -n-Propyl | -n-Butyl | 246 |
| 24 | 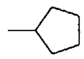 | -n-Propyl | 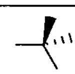 | 310 |
| 25 | 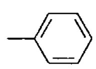 | -n-Propyl | -n-Propyl | 300 Zers. |
| 26 | 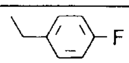 | -n-Propyl | -n-Propyl | 221 |
| 27 | -Ethyl | -n-Propyl | -Ethyl | 229 |
| 28 | -Ethyl | -n-Propyl | 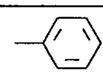 | > 300 Zers. |
| 29 | 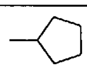 | -n-Propyl | 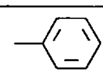 | > 300 Zers. |
| 30 | -Ethyl | -n-Propyl | 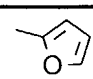 | > 300 Zers. |
| 31 | 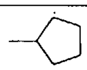 | -n-Propyl | 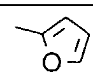 | > 300 Zers. |
| 32 | 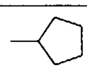 | -n-Butyl | -H | 243 |
| 33 | 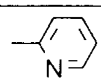 | -Ethyl | 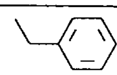 | 287 |
| 34 | 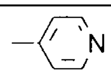 | -Ethyl | 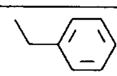 | 346 |
| 35 | 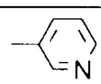 | -Ethyl | 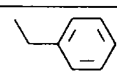 | 331-332 |

| Beispiel Nr. | -$R^2$ | -$R^4$ | -$R^6$ | Schmelzpunkt in °C |
|---|---|---|---|---|
| 36 | 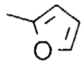 | -n-Propyl | 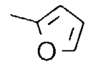 | 385-387 |
| 37 | 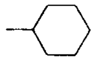 | -Ethyl | 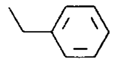 | 260-262 |
| 38 | 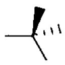 | -Ethyl | 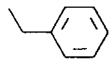 | 243-244 |
| 39 | 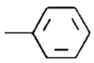 | -Ethyl | 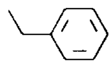 | 343-345 |
| 40 | 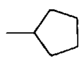 | -Ethyl | 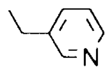 | 261-263 |
| 41 | -Ethyl | -Ethyl | 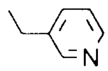 | 225-227 |
| 42 | -Ethyl | -Ethyl | 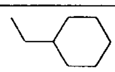 | 230-232 |
| 43 | 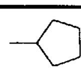 | -Ethyl | 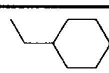 | 273-274 |
| 44 | -Ethyl | -Ethyl | 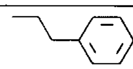 | 282-283 |
| 45 | 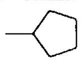 | -Ethyl | 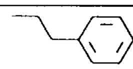 | 292-294 |
| 46 | 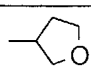 | -n-Propyl | 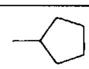 | 289-290 |
| 47 | 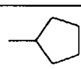 | -n-Propyl | 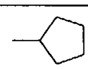 | 306-308 |
| 48 | -Ethyl | -Ethyl | 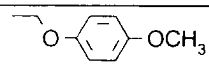 | 225 |
| 49 | 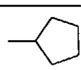 | -Ethyl | 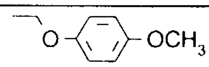 | 234-236 |
| 50 | 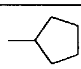 | -Ethyl | 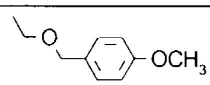 | 182 |
| 51 | 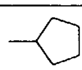 | -Ethyl | 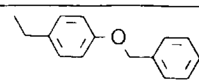 | 251 |
| 52 | 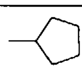 | -Ethyl | 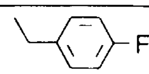 | 252-254 |
| 53 | 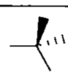 | -Ethyl | 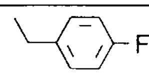 | 262-264 |
| 54 | 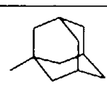 | -Ethyl | 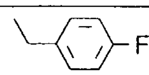 | 337-339 |

| Beispiel Nr. | -$R^2$ | -$R^4$ | -$R^6$ | Schmelz-punkt in °C |
|---|---|---|---|---|
| 55 | 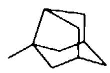 | -Ethyl | 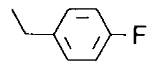 | 325-327 |
| 56 | 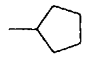 | -Ethyl | 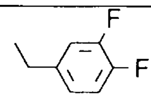 | 264-266 |
| 57 | 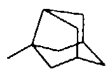 | -Ethyl | 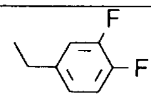 | 315-317 |
| 58 | 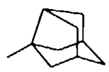 | -Ethyl | 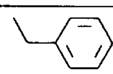 | 320-322 |
| 59 | 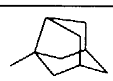 | -Ethyl | 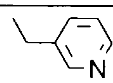 | 330-332 |

a): Methansulfonat

Die Strukturen der zuvor synthetisierten Beispiele der erfindungsgemäßen Verbindungen sind durch NMR-Spektroskopie bestätigt worden.

NMR-spektroskopische Daten ausgewählter Verbindungen:

**Beispiel (1)**

$^1$H-NMR (DMSO-d6): δ = 13.66 (1H, s, broad, NH); 7.34-7.14 (5H, m, Aryl-H); 4.11 (2H, t, J = 7.5 Hz, N-$\underline{CH_2}CH_2CH_3$); 4.07 (2H, s, -$\underline{CH_2}$-Phenyl); 2.79 (2H, qu, J = 7.5 Hz, -$\underline{CH_2}CH_3$); 1.71 (2H, m, N-$CH_2\underline{CH_2}CH_3$); 1.29 (3H, t, J = 7.5 Hz, -$CH_2$-$\underline{CH_3}$); 0.90 (3H, t, J = 7.5 Hz, N-$CH_2CH_2\underline{CH_3}$).

**Beispiel (2)**

$^1$H-NMR (DMSO-d6): δ = 13.70 (1H, s, broad, NH); 7.43-7.14 (5H, m, Aryl-H); 4.09 (2H, t, J = 7.5 Hz, N-$\underline{CH_2}CH_2CH_3$); 4.07 (2H, s, -$\underline{CH_2}$-Phenyl); 2.75 (2H, t, J = 7.5 Hz, -$\underline{CH_2}CH_2CH_3$); 1.74 (4H, m, N-$CH_2\underline{CH_2}CH_3$;-$CH_2\underline{CH_2}CH_3$); 0.92 (6H, m, N-$CH_2CH_2\underline{CH_3}$; -$CH_2CH_2\underline{CH_3}$).

**Beispiel (3)**

$^1$H-NMR (DMSO-d6): δ = 7.34-7.12 (5H, m, Aryl-H); 4.07 (2H, s, $CH_2$-Phenyl); 3.55 (3H, s, N-$CH_3$); 3.23 (1H, m, CH-Cyclopentyl); 2.14-1.52 (8H, m, $CH_2$-Cyclopentyl).

**Beispiel (4)**

$^1$H-NMR (DMSO-d6): δ = 13.68 (1H, s, broad, NH); 7.40-7.14 (5H, m, Aryl-H); 4.19 (2H, qu, J = 7.5 Hz, N-$\underline{CH_2}CH_3$); 4.09 (2H, s, $CH_2$-Aryl); 3.24 (1H, m, CH-Cyclopentyl); 2.26-1.50 (8H, m, $CH_2$-Cyclopentyl); 1.26 (3H, t, J = 7.5 Hz, N-$CH_2\underline{CH_3}$).

**Beispiel (5)**

$^1$H-NMR (DMSO-d6): δ = 13.65 (1H, s, broad, NH); 7.38-7.14 (5H, m, Aryl-H); 4.11 (2H, t, J = 7.5 Hz, N-$\underline{CH_2}CH_2CH_3$); 4.08 (2H, s, Phenyl-$\underline{CH_2}$-); 3.23 (1H, m, CH-Cyclopentyl); 2.14-1.49 (10 H, m, $CH_2$-Cyclopentyl; N-$CH_2\underline{CH_2}CH_3$); 0.89 (3H, t, J = 7.5 Hz, N-$CH_2CH_2\underline{CH_3}$).

**Beispiel (6)**

$^1$H-NMR (DMSO-d6): δ = 13.85 (1H, s, broad, NH); 7.62-7.33 (5H, m, Aryl-H); 4.34 (2H, t, J = 7.5 Hz, N-$\underline{CH_2}$); 4.28 (2H, s, $CH_2$-Phenyl); 3.43 (1H, m, CH-Cyclopentyl); 2.33-1.38 (12H, m, $CH_2$-Cyclopentyl; N-$CH_2$-$\underline{CH_2CH_2}$-$CH_3$); 1.10 (3H, t, J = 7.5 Hz, N-$(CH_2)_3$-$\underline{CH_3}$).

**Beispiel (7)**

$^1$H-NMR (DMSO-d6): δ = 12.58 (1H, s, broad, NH); 7.52-7.14 (10 H, m, Aryl-H); 5.52 (2H, s, N-$CH_2$-Aryl); 4.18 (2H, s, -$CH_2$-Aryl); 3.31 (1H, m, CH-Cyclopentyl); 2.21-1.50 (8H, m, $CH_2$-Cyclopentyl).

**Beispiel (8)**

$^1$H-NMR (DMSO-d6): δ = 13.82 (1H, s, broad, NH); 7.34-7.14 (5H, m, Aryl-H); 4.19 (2H, qu, J = 7.0 Hz, N-$\underline{CH_2}CH_3$); 4.08 (2H, s, -$\underline{CH_2}$-Phenyl); 3.93; 3.43 (4H, 2m, 2 $CH_2$-0); 3.09 (1H, m, -CH-THP); 2.00-1.68 (4H, m, 2-$CH_2$-THP); 1.25 (3H, t, J = 7.0 Hz, N-$CH_2\underline{CH_3}$).

**Beispiel (9)**

$^1$H-NMR (DMSO-d6): δ = 13.84 (1H, s, broad, NH); 7.34-7.14 (5H, m, Aryl-H); 4.18 (2H, qu, J = 7.0 Hz, N-$\underline{CH_2}CH_3$); 4.08 (2H, s, -$\underline{CH_2}$-Phenyl); 4.08-3.74 (4H, m, 2 $CH_2$-O-3THF); 3.62 (1H, m, CH- 3 THF); 2.29 (2H, m, $CH_2$-3 THF); 1.25 (3H, t, J = 7.0 Hz, N $CH_2\underline{CH_3}$).

**Beispiel (10)**

$^1$H-NMR (DMSO-d6): δ = 13.68 (1H, s, broad, NH); 4.12 (2H, t, J = 7.5 Hz, N-$\underline{CH_2}$-$CH_2CH_3$);3.20 (1H, m, CH-Cyclopropyl); 2.45 (3H, s, -$CH_3$); 2.12-1.52 (10 H, m, $CH_2$-Cyclopentyl); 0.89 (3H, t, J = 7.5 Hz, N-$CH_2$-$CH_2\underline{CH_3}$).

**Beispiel (11)**

$^1$H-NMR (DMSO-d6): δ = 13.64 (1H, s, broad, NH); 4.12 (2H, t, J = 7.5 Hz, N-$\underline{CH_2}$-$CH_2CH_3$);3.20 (1H, m, CH-Cyclopentyl); 2.80 (2H, qu, J = 7.5 Hz, -$\underline{CH_2}$-$CH_3$); 2.13-1.52 (10 H, m, $CH_2$-Cyclopentyl; N-$CH_2$-$\underline{CH_2}CH_3$); 1.29 (3H, t, J = 7.5 Hz,-$CH_2$-$\underline{CH_3}$); 0.90 (3H, t, J = 7.5 Hz, -N-$CH_2$-$CH_2\underline{CH_3}$).

**Beispiel (12)**

$^1$H-NMR (DMSO-d6): δ = 13.88 (1H, s, broad, NH); 7.35-7.14 (5H, m, Aryl-H); 4.13 (2H, s, -$\underline{CH_2}$-Phenyl); 4.11 (2H, t, J = 7.5 Hz, N-$\underline{CH_2}CH_2CH_3$); 3.19 (1H, m, CH-Cyclopentyl); 2.09-1.52 (1=H, m, $CH_2$-Cyclopentyl; N-$CH_2\underline{CH_2}CH_3$); 0.89 (3H, t, J = 7.5 Hz, N-$CH_2CH_2\underline{CH_3}$).

**Beispiel (13)**

$^1$H-NMR (DMSO-d6): δ = 13.60 (1H, s, broad, NH); 7.43-7.14 (5H, m, Aryl-H); 4.36 (2H, t, J = 7.5 Hz, N-$\underline{CH_2}$ $(CH_2)_3$-$CH_3$); 4.19 (2H, s, -$\underline{CH_2}$-Phenyl); 3.40 (1H, m, CH-Cyclopentyl); 2.31-1.24 (14H, m, $CH_2$-Cyclopentyl; N-$CH_2$ $(\underline{CH_2})_3$-$CH_3$); 0.89 (3H, m, N-$(CH_2)_4$-$\underline{CH_3}$).

**Beispiel (14)**

$^1$H-NMR (DMSO-d6): δ = 13.85 (1H, s, broad, NH); 8.16 (1H, s, H6); 7.35-7,14 (5H, m, Aryl-H); 4.15 (2H, s, -$\underline{CH_2}$-Phenyl); 3.59 (3H, s, N-$CH_3$).

**Beispiel (15)**

$^1$H-NMR (DMSO-d6): δ = 14.02 (1H, s, broad, NH); 8.19 (1H, s, H6); 7.35-7.14 (5H, m, Aryl-H); 4.18 (2H, s, -$\underline{CH_2}$-Phenyl); 4.15 (2H, t, J = 7.5 Hz, N-$\underline{CH_2}CH_2CH_3$); 1.75 (2H, m, N-$CH_2\underline{CH_2}CH_3$); 0.93 (3H, t, J = 7.5 Hz, N-$CH_2CH_2\underline{CH_3}$).

**Beispiel (16)**

$^1$H-NMR (DMSO-d6): $\delta$ = 13.70 (1H, s, broad, NH); 8.18 (1H, s, H6); 3.59 (3H, s, C-$CH_3$); 3.25 (1H, m, CH-Cyclopentyl); 2.15-1.52 (8H, m, $CH_2$-Cyclopentyl).

**Beispiel (17)**

$^1$H-NMR (DMSO-d6): $\delta$ = 13.70 (1H, s, broad, NH); 8.20 (1H, s, H6); 4.25 (2H, qu, J = 7.0 Hz, N-$\underline{CH_2}$-$CH_3$); 3.27 (1H, m, CH-Cyclopentyl); 2.21-1.52 (8H, m, $CH_2$-Cyclopentyl); 1.29 (3H, t, J = 7.0 Hz, N-$CH_2$-$\underline{CH_3}$).

**Beispiel (18)**

$^1$H-NMR (DMSO-d6): $\delta$ = 13.74 (1H, s, broad, NH); 8.18 (1H, s, H6); 4.14 (2H, t, J = 7.5 Hz, N-$CH_2$-$\underline{CH_2}CH_3$); 3.25 (1H, m, CH-Cyclopentyl); 2.15-1.54 (10 H, m, $CH_2$-Cyclopentyl; N-$CH_2$-$\underline{CH_2}CH_3$); 0.90 (3H, t, J = 7.5 Hz, N-$CH_2$-$CH_2\underline{CH_3}$).

**Beispiel (19)**

$^1$H-NMR (DMSO-d6): $\delta$ = 13.62 (1H, s, broad, NH); 4.11 (2H, t, J = 7.5 Hz, N-$\underline{CH_2}CH_2CH_3$); 3.23 (1H, m, CH-Cyclopentyl); 2.37 (3H, s, -$CH_3$); 2.14-1.50 (10 H, m, $CH_2$-Cyclopentyl, N-$CH_2\underline{CH_2}CH_3$); 0.90 (3H, t, J = 7.5 Hz, N-$CH_2CH_2\underline{CH_3}$).

**Beispiel (20)**

$^1$H-NMR (DMSO-d6): $\delta$ = 13.62 (1H, s, broad, NH); 4.12 (2H, t, J = 7.5 Hz, N-$\underline{CH_2}CH_2CH_3$); 3.25 (1H, m, CH-Cyclopentyl); 2.74 (2H, qu, J = 7.5 Hz, -$\underline{CH_2}CH_3$); 2.17-1.51 (10 H, m, $CH_2$-Cyclopentyl; N-$CH_2\underline{CH_2}CH_3$); 1.29 (3H, t, J = 7.5 Hz,-$CH_2\underline{CH_3}$); 0.90 (3H, t, J = 7.5 Hz, N-$CH_2CH_2\underline{CH_3}$).

**Beispiel (21)**

$^1$H-NMR (DMSO-d6): $\delta$ = 13.58 (1H, s, broad, NH); 4.12 (2H, t, J = 7.5 Hz, N-$\underline{CH_2}CH_2CH_3$); 3.24 (1H, m, CH-Cyclopentyl);2.69 (2H, t, J = 7.5 Hz, -$\underline{CH_2}CH_2CH_3$); 2.15-1.50 (12H, m, $CH_2$-Cyclopentyl; $(CH_2\underline{CH_2}CH_3)_2$); 0.95; 0.90 (6H, 2t, J = 7.5 Hz, $(CH_2CH_2\underline{CH_3})_2$).

**Beispiel (22)**

$^1$H-NMR (DMSO-d6): $\delta$ = 13.62 (1H, s, broad, NH); 4.13 (2H, t, J = 7.5 Hz, N-$\underline{CH_2}CH_2CH_3$); 3.24 (1H, m, CH-Cyclopentyl); 3.05 (1H, m, CH-Isoprop.); 2.14-1.50 (10 H, m, $CH_2$-Cyclopentyl; N-$CH_2\underline{CH_2}CH_3$); 1.32 (6H, d, J = 7.5 Hz, $CH_3$-Isoprop.); 0.90 (3H, t, J = 7.5 Hz, $CH_2CH_2\underline{CH_3}$).

**Beispiel (23)**

$^1$H-NMR (DMSO-d6): $\delta$ = 13.62 (1H, s, broad, NH); 4.12 (2H, t, J = 7.5 Hz, N-$\underline{CH_2}CH_2CH_3$); 3.24 (1H, m, Cyclopentyl-H); 2.70 (2H, t, J = 7.5 Hz, $\underline{CH_2}$-$CH_2CH_2CH_3$); 2.15-1.26 (14H, m, $CH_2$-Cyclopentyl; N-$CH_2\underline{CH_2}$-$CH_3$, $CH_2$-$\underline{CH_2CH_2}CH_3$); 0.90 (6H, m, N-$CH_2CH_2CH_3$); $(-CH_2)_3$-$\underline{CH_3}$).

**Beispiel (24)**

$^1$H-NMR ($CDCl_3$): $\delta$ = 4.36 (2H, t, J = 7.5 Hz, N-$\underline{CH_2}CH_2CH_3$); 3.40 (1H, m, CH -Cyclopentyl); 2.32-1.63 (10 H, m, $CH_2$-Cyclopentyl; N-$CH_2\underline{CH_2}CH_3$); 1.46 (9H, s, $C(CH_3)_3$; 1.02 (3H, t, J = 7.5 Hz, N-$CH_2CH_2CH_3$).

**Beispiel (25)**

$^1$H-NMR (DMSO-d6): $\delta$ = 14.28 (1H, s, broad, NH); 8.26-7.45 (5H, m, Aryl-H); 4.14 (2H, t, J = 7.5 Hz, N-$\underline{CH_2}CH_2CH_3$); 2.71 (2H, t, J = 7.5 Hz, -$\underline{CH_2}CH_2CH_3$); 1.76 (4H, m, $(-CH_2\underline{CH_2}CH_3)_2$); 0.98; 0.93 (6H, 2t, $(-CH_2CH_2\underline{CH_3})_2$).

**Beispiel (26)**

$^1$H-NMR (DMSO-d6): δ = 7.44-7.05 (4H, m, Aryl-H); 4.13 (2H, s, $CH_2$-Phenyl); 4.11 (2H, t, J = 7.5 Hz, N-$\underline{CH_2}CH_2$-$CH_3$); 2.68 (2H, t, J = 7.5 Hz, -$\underline{CH_2}CH_2$-$CH_3$); 1.74 (4H, m, (-$CH_2\underline{CH_2}$-$CH_3)_2$); 0.95; 0.89 (6H, 2t, J = 7.5 Hz, $(CH_2CH_2$-$\underline{CH_3})_2$).

**Beispiel (27)**

$^1$H-NMR (DMSO-d6): δ = 13.64 (1H, s, broad, NH); 4.12 (2H, t, J = 7.5 Hz; N-$\underline{CH_2}CH_2CH_3$); 2.76 (4H, m, $(\underline{CH_2}CH_3)_2$); 1.73 (2H, m, N-$CH_2\underline{CH_2}CH_3$)); 2.29 (6H, m, $(CH_2$-$\underline{CH_3})_2$); 0.90 (3H, t, J = 7.5 Hz, N-$CH_2CH_2\underline{CH_3}$).

**Beispiel (28)**

$^1$H-NMR (DMSO-d6): δ = 13.78 (1H, s, broad, NH); 8.19-7.43 (5H, m, Aryl-H); 4.21 (2H, t, J = 7.5 Hz, N-$\underline{CH_2}CH_2CH_3$); 2.83 (2H, qu, J = 7.5 Hz, -$\underline{CH_2}CH_3$); 1.81 (2H, m, N-$CH_2\underline{CH_2}CH_3$); 1.33 (3H, t, j = 7.5 Hz, -$CH_2\underline{CH_3}$); 0.95 (3H, t, J = 7.5 Hz, -N-$CH_2CH_2\underline{CH_3}$).

**Beispiel (29)**

$^1$H-NMR (DMSO-d6): δ = 13.70 (1H, s, broad, NH); 8.19-7.38 (5H, m, Aryl-H); 4.21 (2H, t, J = 7.5 Hz, N-$\underline{CH_2}CH_2CH_3$); 3.28 (1H, m, CH-Cyclopentyl); 2.19-1.50 (10 H, m, $CH_2$-Cyclopentyl; N-$CH_2\underline{CH_2}CH_3$); 0.94 (3H, t, J = 7.5 Hz, N-$CH_2CH_2\underline{CH_3}$).

**Beispiel (30)**

$^1$H-NMR (DMSO-d6): δ = 13.56 (1H, s, broad, NH); 7.70; 6.91; 6.50 (3H, 3m, Furyl-H); 4.00 (2H, t, J = 7.5 Hz, N-$\underline{CH_2}CH_2CH_3$); 2.66 (2H, qu, J = 7.5 Hz, -$\underline{CH_2}CH_3$); 1.61 (2H, m, N-$CH_2$-$\underline{CH_2}$-$CH_3$); 1.14 (3H, t, J = 7.5 Hz, N-$CH_2CH_2\underline{CH_3}$); 0.77 (3H, t, J = 7.5 Hz, -$CH_2\underline{CH_3}$).

**Beispiel (31)**

$^1$H-NMR (DMSO-d6): δ = 13.84 (1H, s, broad, NH); 8.00; 7.18; 6.78 (3H, 3m, Furan-H); 4.26 (2H, t, J = 7.5 Hz, N-$\underline{CH_2}CH_2CH_3$); 3.37 (1H, m, CH-Cyclopentyl); 2.28-1.62 (10 H, m, $CH_2$-Cyclopentyl; N-$CH_2\underline{CH_2}CH_3$); 1.04 (3H, t, J = 7.5 Hz; N-$CH_2CH_2\underline{CH_3}$).

Die nachfolgende Tabelle enthält $KiA_1$ (human) und $KiA_2$ (Ratte) Rezeptorbindungswerte.

Tabelle 9:

| Beispiel-Nr.: | $K_iA_1$ [nM] | $K_iA_2$ [nM] |
|---|---|---|
| 2 | 8,1 | 158 |
| 3 | 1,9 | 363 |
| 4 | 1,4 | 422 |
| 5 | 1,7 | 730 |
| 6 | 8,3 | 345 |
| 13 | 10,3 | 1231 |
| 17 | 81,5 | 3292 |
| 21 | 5,8 | 731 |
| 22 | 6,4 | 307 |
| 23 | 6,4 | 532 |
| 24 | 6,0 | 539 |
| 31 | 11,4 | 4455 |

Die nachfolgende Tabelle enthält $KiA_3$ (human) Rezeptorbindungswerte.

Tabelle 10:

| Beispiel Nr. | $K_iA_3$ [nM] |
|---|---|
| 1 | 2 |
| 7 | 20 |
| 11 | 2 |
| 13 | 26 |
| 23 | 22 |
| 38 | 30 |
| 44 | 5,3 |
| 48 | 25 |

Die erfindungsgemäßen Verbindungen können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Eine therapeutisch wirksame Tagesdosis beträgt zwischen 1 und 800 mg, bevorzugt 10 - 300 mg pro Erwachsener.

Die nachfolgenden Beispiele illustrieren die vorliegende Erfindung ohne sie jedoch in ihrem Umfang zu beschränken:

<u>Pharmazeutische Formulierungsbeispiele</u>

| A) | Tabletten | pro Tablette |
|---|---|---|
| | Wirkstoff<br>Milchzucker<br>Maisstärke | 100 mg<br>140 mg<br>240 mg |

(fortgesetzt)

| A) | Tabletten | pro Tablette |
|---|---|---|
| | Polyvinylpyrrolidon<br>Magnesiumstearat | 15 mg<br>5 mg<br>500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff<br>Maisstärke<br>Milchzucker<br>Mikrokristalline Cellulose<br>Polyvinylpyrrolidon<br>Natrium-carboxymethylstärke<br>Magnesiumstearat | 80 mg<br>190 mg<br>55 mg<br>35 mg<br>15 mg<br>23 mg<br>2 mg<br>400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxymethylstärke und das Magnesiumstearat, vermischt und verpreßt das Gemisch zu Tabletten geeigneter Größe.

| C) | Dragées | pro Dragée |
|---|---|---|
| | Wirkstoff<br>Maisstärke<br>Milchzucker<br>Polyvinylpyrrolidon<br>Magnesiumstearat | 5 mg<br>41,5 mg<br>30 mg<br>3 mg<br>0,5 mg<br>80 mg |

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichten aus Zucker und Talkum besteht. Die fertigen Dragées werden mit Wachs poliert.

| D) | Kapseln | pro Kapsel |
|---|---|---|
| | Wirkstoff<br>Maisstärke<br>Magnesiumstearat | 50 mg<br>268,5 mg<br>1,5 mg<br>320 mg |

Substanz und Maisstärke werden gemischt und mit Wasser befeuchtet. Die feuchte Masse wird gesiebt und getrocknet. Das trockene Granulat wird gesiebt und mit Magensiumstearat gemischt. Die Endmischung wird in Hartgelatinekapseln Größe 1 abgefüllt.

| E) | Ampullenlösung | |
|---|---|---|
| | Wirkstoff<br>Natriumchlorid<br>Aqua pro inj. | 50 mg<br>50 mg<br>5 ml |

Der Wirkstoff wird bei Eigen-pH oder gegebenenfalls bei pH 5,5 bis 6,5 in Wasser gelöst und mit Natriumchlorid als Isotonans versetzt. die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| F) | Suppositorien | |
|---|---|---|
| | Wirkstoff<br>Adeps solidus | 50 mg<br>1650 mg<br>1700 mg |

Das Hartfett wird geschmolzen. Bei 40°C wird die gemahlene Wirksubstanz homogen dispergiert. Es wird auf 38°C abgekühlt und in schwach vorgekühlte Suppositorienformen ausgegossen.

| G) | orale Suspension | |
|---|---|---|
| | Wirkstoff<br>Hydroxyethylcellulose<br>Sorbinsäure<br>Sorbit (70%ig)<br>Glycerin<br>Aroma<br>Wasser ad | 50 mg<br>50 mg<br>5 mg<br>600 mg<br>200 mg<br>15 mg<br>5 ml |

Destilliertes Wasser wird auf 70°C erhitzt. Hierin wird unter Rühren Hydroxyethylcellulose gelöst. Nach Zugabe von Sorbitlösung und Glycerin wird auf Raumtemperatur abgekühlt. Bei Raumtemperatur werden Sorbinsäure, Aroma und Substanz zugegeben. Zur Entlüftung der Suspension wird unter Rühren evakuiert.

**Patentansprüche**

**1.** Verbindungen erhältlich durch folgende Reaktionsschritte:

a) Umsetzung von 0,1 Mol eines Triazols der allgemeinen Formel (3)

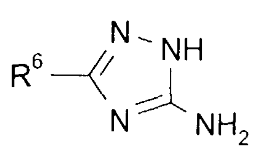

(3)

worin

$R^6$ Wasserstoff, Methyl, Ethyl, n-Propyl, i-Propyl, n-Butyl, tert.-Butyl, Phenyl, Benzyl, Cyclopentyl, 2-Furyl, 3-Pyridylmethyl, Cyclohexylmethyl, 2-Phenylethyl, (4-MeO-Ph)-O-$CH_2$-, (4-MeO-Ph)-$CH_2$-O-$CH_2$-, (4-$PhCH_2O$-Ph)-$CH_2$-, 4-F-Ph-$CH_2$- oder 3,4-F-Ph-$CH_2$- bedeuten können,

mit 0,13 Mol Cyanessigsäureethylester in Gegenwart von 0,1 Mol Natriumethylat in absolutem Ethanol unter Rückfluß über einen Zeitraum von 4-6 h zu dem entsprechenden Triazolopyrimidin, welches nach Abkühlen des Reaktionsgemisches sowie anschließendem Versetzen mit Wasser und Ansäuern isoliert wird

b) anschließende Umsetzung von 66mMol des so erhaltenen Triazolopyrimidins bei Raumtemperatur mit 76mMol eines Alkylhalogenids des Typs R'-X, wobei R' für den Rest $R^4$ steht, wobei $R^4$ Methyl, Ethyl, n-Propyl, n-Butyl, n-Pentyl oder Benzyl bedeutet, in wasserfreiem Dimethylformamid in Gegenwart von 76mMol Kaliumcarbonat über einen Zeitraum von 1-2 Tagen zu dem entsprechenden alkylierten Triazolopyrimidin, das nach dessen Isolierung gegebenenfalls einer chromatographischen Reinigung unterworfen wird,

c) Umsetzung von 10mMol des aus Schritt b) resultierenden Alkyltriazolopyrimidins mit 20 mMol Isoamylnitrit bei einer Temperatur von <0°C in wasserfreiem Dimethylformamid über einen Zeitraum von 4-24 h zu der entsprechenden Nitrosoverbindung, die isoliert wird,

d1) Suspendieren von 12,7 mMol eines gemäß Schritt c) erhaltenen Nitrosoderivats in Wasser, Zusetzen von 4,9 g $Na_2S_2O_4$, Erhitzen der Suspension auf 80°C, Zugabe von 15 ml 50%iger $H_2SO_4$ und Kochen der Mischung unter Rückfluß bis zum vollständigen Umsatz, wodurch nach Abkühlen das Produkt entweder dirket als ausfallendes Semisulfat oder, nach Neutralisieren mit 30%-iger Natronlauge, als ausgefallene freie Base isoliert werden kann,

oder

d2) Lösen von 11,5 mMol eines gemäß Schritt c) erhaltenen Nitrosoderivats in einer Mischung aus 175 ml 25%-igem wässrigen Ammoniak und 36 ml Ethanol und Umsetzung mit einer Lösung von 6,1 g $Na_2S_2O_4$ in 57 ml Wasser bei Raumtemperatur über einen Zeitraum von 1,5-3 h, Abfiltrieren des resultierenden Diamins,

e) Versetzen von 30 mMol eines gemäß Schritt d1) oder gemäß Schritt d2) erhaltenen, in wasserfreiem Diemthylformamid suspendierten, Diamins mit 45 mMol Dimethylaminopyridin (im Falle von Semisulfaten mit 78 mMol) und Rühren der Mischung über einen Zeitraum von 30 Minuten bei Raumtemperatur, Abkühlen des Reaktionsgemisches auf eine Temperatur von 5-10°C und Zugabe von in 16 ml wasserfreiem Dimethylformamid gelösten 39 mMol eines Carbonsäurechlorids des Typs $R^2$-COCl,
wobei

$R^2$ Methyl, Ethyl, n-Propyl, tert-Butyl, Phenyl, Benzyl, 2-Furyl, 2-Pyridyl, 3-Pyridyl, 4-Pyridyl, 3-Tetrahydrofuranyl, 4-Tetrahydropyranyl, Cyclopentyl, Cyclohexyl, 4-F-Ph-$CH_2$-, Adamantan-1-yl oder Noradamantan-3-yl bedeuten,

sowie 2-stündiges Rühren bei 10°C und anschließendes Rühren über Nacht bei Raumtemperatur, woraus die entsprechenden Amide resultieren, die nach Aufarbeitung und Reinigung durch Kristallisation oder Chromatographie isoliert werden,

f) Cyclisieren von 14,4 mMol der in Schritt e) erhaltenen Carbonsäureamiderivate durch Erhitzen der Amide in einem Lösemittelgemisch aus 75,5 ml Wasser und 37,8 ml Ethanol nach Zugabe von 17,4 ml 50%iger NaOH und 4,82 g $Ca(OH)_2$ über einen Zeitraum von 24 h bei Rückflußtemperatur und Isolieren der entsprechenden Cyclisierungsprodukte nach Abkühlen und Ansäuern des Reaktionsgemisches sowie gegebenenfalls Reinigung durch Umkristallisation oder Chromatographie.

**2.** Verbindung erhältlich gemäß Anspruch 1, in denen $R^2$ Cyclopentyl bedeutet, ausgewählt aus der Gruppe bestehend aus:

- Verbindung vom Schmp. 250°C, worin $R^6$ H und $R^4$ n-Propyl bedeuten;
- Verbindung vom Schmp. 262-264°C, worin $R^6$ -Methyl und $R^4$ n-Propyl bedeuten;
- Verbindung vom Schmp. 253°C, worin $R^6$ -Ethyl und $R^4$ n-Propyl bedeuten;
- Verbindung vom Schmp. 256°C, worin $R^6$ -n-Propyl und $R^4$ n-Propyl bedeuten;
- Verbindung vom Schmp. 260-261°C, worin $R^6$ -i-Propyl und $R^4$ n-Propyl bedeuten;
- Verbindung vom Schmp. 246°C, worin $R^6$ -n-Butyl und $R^4$ n-Propyl bedeuten;
- Verbindung vom Schmp. 310°C, worin $R^6$ -t-Butyl und $R^4$ n-Propyl bedeuten;
- Verbindung vom Schmp. >300°C (Zers.), worin $R^6$ 2-Furyl und $R^4$ n-Propyl bedeuten;

- Verbindung vom Schmp. 270°C, worin $R^6$ Benzyl und $R^4$ Benzyl bedeuten;
- Verbindung vom Schmp. 265-267°C, worin $R^6$ Benzyl und $R^4$ Methyl bedeuten;
- Verbindung vom Schmp. 242°C, worin $R^6$ Benzyl und $R^4$ Ethyl bedeuten;
- Verbindung vom Schmp. 234-235°C, worin $R^6$ Benzyl und $R^4$ n-Propyl bedeuten;
- Verbindung vom Schmp. 238-240°C, worin $R^6$ Benzyl und $R^4$ n-Butyl bedeuten;
- Verbindung vom Schmp. 231-236°C, worin $R^6$ Benzyl und $R^4$ n-Pentyl bedeuten;
- Verbindung vom Schmp. >300°C (Zers.), worin $R^6$ Phenyl und $R^4$ n-Propyl bedeuten;
- Verbindung vom Schmp. 217-220°C, worin $R^6$ H und $R^5$ n-Propyl bedeuten;
- Verbindung vom Schmp. 260°C, worin $R^6$ H und $R^5$ Ethyl bedeuten;
- Verbindung vom Schmp. 261-263°C, worin $R^6$ 3-Pyridylmethyl und $R^4$ Ethyl bedeuten;
- Verbindung vom Schmp. 273-274°C, worin $R^6$ Cyclohexylmethyl und $R^4$ Ethyl bedeuten;
- Verbindung vom Schmp. 292-294°C, worin $R^6$ 2-Phenylethyl und $R^4$ Ethyl bedeuten;
- Verbindung vom Schmp. 306-308°C, worin $R^6$ Cyclopentyl und $R^4$ n-Propyl bedeuten;
- Verbindung vom Schmp. 234-236°C, worin $R^6$ (4-MeO-Ph)-$OCH_2$- und $R^4$ Ethyl, bedeuten;
- Verbindung vom Schmp. 182°C, worin $R^6$ (4-MeO-Ph)-$CH_2$-O-$CH_2$- und $R^4$ Ethyl, bedeuten;
- Verbindung vom Schmp. 251°C, worin $R^6$ (4-$PhCH_2O$-Ph)-$CH_2$- und $R^4$ Ethyl bedeuten;
- Verbindung vom Schmp. 252-254°C, worin $R^6$ 4-Fluorbenzyl und $R^4$ Ethyl bedeuten;
- Verbindung vom Schmp. 264-266°C, worin $R^6$ 3,4-Difluorbenzyl und $R^4$ Ethyl bedeuten;

gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**3.** Verbindung erhältlich gemäß Anspruch 1, in denen $R^2$ Ethyl bedeutet, ausgewählt aus der Gruppe bestehend aus:

- Verbindung vom Schmp. 229°C, worin $R^6$ -Ethyl und $R^4$ n-Propyl bedeuten;
- Verbindung vom Schmp. 245-248°C, worin $R^6$ Cyclopentyl und $R^4$ n-Propyl bedeuten;
- Verbindung vom Schmp. >300°C (Zers.), worin $R^6$ 2-Furyl und $R^4$ n-Propyl bedeuten;
- Verbindung vom Schmp. 223°C, worin $R^6$ Benzyl und $R^4$ n-Propyl bedeuten;
- Verbindung vom Schmp. >300°C (Zers.), worin $R^6$ Phenyl und $R^4$ n-Propyl bedeuten;
- Verbindung vom Schmp. 225-227°C, worin $R^6$ 3-Pyridylmethyl und $R^4$ Ethyl bedeuten;
- Verbindung vom Schmp. 230-232°C, worin $R^6$ Cyclohexylmethyl und $R^4$ Ethyl bedeuten;
- Verbindung vom Schmp. 282-283°C, worin $R^6$ 2-Phenylethyl und $R^4$ Ethyl bedeuten;
- Verbindung vom Schmp. 225°C, worin $R^6$ (4-MeO-Ph)-$OCH_2$- und $R^4$ Ethyl bedeuten;

gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**4.** Verbindung erhältlich gemäß Anspruch 1, in denen $R^2$ Benzyl bedeutet, ausgewählt aus der Gruppe bestehend aus:

- Verbindung vom Schmp. 306 °C, worin $R^6$ H und $R^4$ Methyl bedeuten;
- Verbindung vom Schmp. 222-223°C, worin $R^6$ H und $R^4$ n-Propyl bedeuten;
- Verbindung vom Schmp. 233°C, worin $R^6$ Cyclopentyl und $R^4$ n-Propyl bedeuten;

gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**5.** Verbindung erhältlich gemäß Anspruch 1, in denen $R^2$ Noradamantan-3-yl bedeutet, ausgewählt aus der Gruppe bestehend aus:

- Verbindung vom Schmp. 325-327°C, worin $R^6$ 4-Fluorbenzyl und $R^4$ Ethyl bedeuten;
- Verbindung vom Schmp. 315-317°C, worin $R^6$ 3,4-Difluorbenzyl und $R^4$ Ethyl bedeuten;
- Verbindung vom Schmp. 320-322°C, worin $R^6$ Benzyl und $R^4$ Ethyl bedeuten;
- Verbindung vom Schmp. 330-332°C, worin $R^6$ 3-Pyridylmethyl und $R^4$ Ethyl bedeuten,

gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**6.** Verbindung erhältlich gemäß Anspruch 1, ausgewählt aus der Gruppe bestehend aus:

- Verbindung vom Schmp. 300°C (Zers.), worin $R^6$ -n-Propyl, $R^4$ n-Propyl, $R^2$ Phenyl bedeuten;
- Verbindung vom Schmp. 221°C, worin $R^6$ -n-Propyl, $R^4$ n-Propyl, $R^2$ 4-Fluorbenzyl bedeuten;
- Verbindung vom Schmp. 248-250°C, worin $R^6$ Cyclopentyl, $R^4$ n-Propyl, $R^2$ Methyl bedeuten;
- Verbindung vom Schmp. 227°C, worin $R^6$ Benzyl, $R^4$ Ethyl, $R^2$ 3-Tetrahydrofuryl bedeuten;
- Verbindung vom Schmp. 237°C, worin $R^6$ Benzyl, $R^4$ Ethyl, $R^2$ 4-Tetrahydropyranyl bedeuten;
- Verbindung vom Schmp. 287°C, worin $R^6$ Benzyl, $R^4$ Ethyl, $R^2$ 2-Pyridyl bedeuten;
- Verbindung vom Schmp. 346°C, worin $R^6$ Benzyl, $R^4$ Ethyl, $R^2$ 4-Pyridyl bedeuten;
- Verbindung vom Schmp. 202°C, worin $R^6$ Benzyl, $R^4$ n-Propyl, $R^2$ n-Propyl bedeuten;
- Verbindung vom Schmp. 331-332,°C, worin $R^6$ Benzyl, $R^4$ Ethyl, $R^2$ 3-Pyridyl bedeuten;
- Verbindung vom Schmp. 260-262°C, worin $R^6$ Benzyl, $R^4$ Ethyl, $R^2$ Cyclohexyl bedeuten;
- Verbindung vom Schmp. 385-387°C, worin $R^6$ 2-Furyl, $R^4$ n-Propyl, $R^2$ 2-Furyl bedeuten;
- Verbindung vom Schmp. 289-290°C, worin $R^6$ Cyclopentyl, $R^4$ n-Propyl, $R^2$ 3-Tetrahydrofuryl bedeuten;
- Verbindung vom Schmp. 262-264°C, worin $R^6$ 4-Fluorbenzyl, $R^4$ Ethyl, $R^2$ tert.-Butyl bedeuten;
- Verbindung vom Schmp. 337-339°C, worin $R^6$ 4-Fluorbenzyl, $R^4$ Ethyl, $R^2$ Adamantan-1-yl bedeuten;

gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**7.** Verwendung einer Verbindung gemäß einem der Ansprüche 1 bis 6 zur Herstellung eines Arzneimittels.

**8.** Pharmazeutische Zubereitungen, enthaltend als Wirkstoff eine oder mehrere Verbindungen gemäß einem der Ansprüche 1 bis 6 oder deren physiologisch verträgliche Säureadditionssalze in Kombination mit üblichen Hilfs- und/oder Trägerstoffen.

## Claims

**1.** Compounds obtainable by the following reaction steps:

a) reacting 0.1 mol of a triazole of general formula (3)

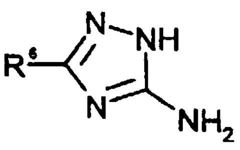

(3)

wherein

$R^6$ may denote hydrogen, methyl, ethyl, n-propyl, isopropyl, n-butyl, tert.-butyl, phenyl, benzyl, cyclopentyl, 2-furyl, 3-pyridylmethyl, cyclohexylmethyl, 2-phenylethyl, (4-MeO-Ph)-O-$CH_2$-, (4-MeO-Ph) -$CH_2$-O-$CH_2$-, (4-$PhCH_2O$-Ph) -$CH_2$-, 4-F-Ph-$CH_2$-or 3,4-F-Ph-$CH_2$-,

with 0.13 mol of ethyl cyanoacetate in the presence of 0.1 mol of sodium ethoxide in absolute ethanol at reflux temperature over a period of 4 to 6 hours to obtain the corresponding triazolopyrimidine, which is isolated after the reaction mixture has been cooled and then mixed with water and acidified,

b) subsequently reacting 66 mMol of the triazolopyrimidine thus obtained at ambient temperature with 76 mMol of an alkyl halide of the R'-X type, wherein R' denotes the group $R^4$, where

$R^4$ denotes methyl, ethyl, n-propyl, n-butyl, n-pentyl or benzyl,

in anhydrous dimethylformamide in the presence of 76 mMol of potassium carbonate over a period of 1 to 2

days to obtain the corresponding alkylated triazolopyrimidine which after being isolated is optionally subjected to chromatographic purification,

c) reacting 10 mMol of the alkyl triazolopyrimidine resulting from step b) with 20 mMol of isoamyl nitrite at a temperature of <0°C in anhydrous dimethylformamide over a period of 4 to 24 hours to obtain the corresponding nitroso compound which is isolated,

d1) suspending 12.7 mMol of a nitroso derivative obtained according to step c) in water, adding 4.9 g of $Na_2S_2O_4$, heating the suspension to 80°C, adding 15 ml of 50% $H_2SO_4$ and refluxing the mixture until the reaction is complete, as a result of which, after cooling, the product can be isolated either directly as a precipitated semisulphate or, after neutralisation with 30% sodium hydroxide solution, as a precipitated free base,

or

d2) dissolving 11.5 mMol of a nitroso derivative obtained according to step c) in a mixture of 175 ml of 25% aqueous ammonia and 36 ml of ethanol and reacting with a solution of 6.1 g of $Na_2S_2O_4$ in 57 ml of water at ambient temperature over a period of 1.5 to 3 hours, filtering off the resulting diamine,

e) combining 30 mMol of a diamine obtained according to step d1) or d2), suspended in anhydrous dimethylformamide, with 45 mMol of dimethylaminopyridine (with 78 mMol in the case of semisulphates) and stirring the mixture for a period of 30 minutes at ambient temperature, cooling the reaction mixture to a temperature of 5 to 10°C and adding 39 mMol of a carboxylic acid chloride of the type $R^2$-COCl, dissolved in 16 ml of anhydrous dimethylformamide, wherein

$R^2$ denotes methyl, ethyl, n-propyl, tert.-butyl, phenyl, benzyl, 2-furyl, 2-pyridyl, 3-pyridyl, 4-pyridyl, 3-tetrahydrofuranyl, 4-tetrahydropyranyl, cyclopentyl, cyclohexyl, 4-F-Ph-$CH_2$-, adamantan-1-yl or noradamantan-3-yl,

and stirring for 2 hours at 10°C and subsequently stirring overnight at ambient temperature, resulting in the corresponding amides which are isolated after working up and purification by crystallisation or chromatography,

f) cyclising 14.4 mMol of the carboxylic acid amide derivative obtained in step e) by heating the amides in a solvent mixture of 75.5 ml of water and 37.8 ml of ethanol with the addition of 17.4 ml of 50% NaOH and 4.82 g of $Ca(OH)_2$, for a period of 24 hours at reflux temperature and isolating the corresponding cyclisation products after cooling and acidifying the reaction mixture and optionally purifying it by recrystallisation or chromatography.

**2.** Compound obtainable according to claim 1 wherein $R^2$ denotes cyclopentyl, selected from the group consisting of:

- Compound of melting point 250°C wherein $R^6$ denotes H and $R^4$ denotes n-propyl;
- Compound of melting point 262-264°C wherein $R^6$ denotes methyl and $R^4$ denotes n-propyl;
- Compound of melting point 253°C wherein $R^6$ denotes ethyl and $R^4$ denotes n-propyl;
- Compound of melting point 256°C wherein $R^6$ denotes n-propyl and $R^4$ denotes n-propyl;
- Compound of melting point 260-261°C wherein $R^6$ denotes isopropyl and $R^4$ denotes n-propyl;
- Compound of melting point 246°C wherein $R^6$ denotes n-butyl and $R^4$ denotes n-propyl;
- Compound of melting point 310°C wherein $R^6$ denotes t-butyl and $R^4$ denotes n-propyl;
- Compound of melting point >300°C (decomp.) wherein $R^6$ denotes 2-furyl and $R^4$ denotes n-propyl;
- Compound of melting point 270°C wherein $R^6$ denotes benzyl and $R^4$ denotes benzyl;
- Compound of melting point 265-267°C wherein $R^6$ denotes benzyl and $R^4$ denotes methyl;
- Compound of melting point 242°C wherein $R^6$ denotes benzyl and $R^4$ denotes ethyl;
- Compound of melting point 234-235°C wherein $R^6$ denotes benzyl and $R^4$ denotes n-propyl;
- Compound of melting point 238-240°C wherein $R^6$ denotes benzyl and $R^4$ denotes n-butyl;
- Compound of melting point 231-236°C wherein $R^6$ denotes benzyl and $R^4$ denotes n-pentyl;
- Compound of melting point >300°C (decomp.), wherein $R^6$ denotes phenyl and $R^4$ denotes n-propyl;
- Compound of melting point 217-220°C wherein $R^6$ denotes H and $R^5$ denotes n-propyl;
- Compound of melting point 260°C wherein $R^6$ denotes H and $R^5$ denotes ethyl;
- Compound of melting point 261-263°C wherein $R^6$ denotes 3-pyridylmethyl and $R^4$ denotes ethyl;
- Compound of melting point 273-274°C wherein $R^6$ denotes cyclohexylmethyl and $R^4$ denotes ethyl;

- Compound of melting point 292-294°C wherein $R^6$ denotes 2-phenylethyl and $R^4$ denotes ethyl;
- Compound of melting point 306-308°C wherein $R^6$ denotes cyclopentyl and $R^4$ denotes n-propyl;
- Compound of melting point 234-236°C wherein $R^6$ denotes (4-MeO-Ph)-$OCH_2$- and $R^4$ denotes ethyl;
- Compound of melting point 182°C wherein $R^6$ denotes (4-MeO-Ph)-$CH_2$-O-$CH_2$- and $R^4$ denotes ethyl;
- Compound of melting point 251°C wherein $R^6$ denotes (4-$PhCH_2O$-Ph)-$CH_2$- and $R^4$ denotes ethyl;
- Compound of melting point 252-254°C wherein $R^6$ denotes 4-fluorobenzyl and $R^4$ denotes ethyl;
- Compound of melting point 264-266°C wherein $R^6$ denotes 3,4-difluorobenzyl and $R^4$ denotes ethyl;

optionally in the form of the racemates, the enantiomers, the diastereomers and the mixtures thereof and optionally the pharmacologically harmless acid addition salts thereof.

**3.** Compound obtainable according to claim 1 wherein $R^2$ denotes ethyl, selected from the group consisting of:

- Compound of melting point 229°C wherein $R^6$ denotes ethyl and $R^4$ denotes n-propyl;
- Compound of melting point 245-248°C wherein $R^6$ denotes cyclopentyl and $R^4$ denotes n-propyl;
- Compound of melting point >300°C (decomp.) wherein $R^6$ denotes 2-furyl and $R^4$ denotes n-propyl;
- Compound of melting point 223°C wherein $R^6$ denotes benzyl and $R^4$ denotes n-propyl;
- Compound of melting point >300°C (decomp.) wherein $R^6$ denotes phenyl and $R^4$ denotes n-propyl;
- Compound of melting point 225-227°C wherein $R^6$ denotes 3-pyridylmethyl and $R^4$ denotes ethyl;
- Compound of melting point 230-232°C wherein $R^6$ denotes cyclohexylmethyl and $R^4$ denotes ethyl;
- Compound of melting point 282-283°C wherein $R^6$ denotes 2-phenylethyl and $R^4$ denotes ethyl;
- Compound of melting point 225°C wherein $R^6$ denotes (4-MeO-Ph)-$OCH_2$- and $R^4$ denotes ethyl;

optionally in the form of their racemates, the enantiomers, the diastereomers and the mixtures thereof and optionally the pharmacologically harmless acid addition salts thereof.

**4.** Compound obtainable according to claim 1 wherein $R^2$ denotes benzyl, selected from the group consisting of:

- Compound of melting point 306°C wherein $R^6$ denotes H and $R^4$ denotes methyl;
- Compound of melting point 222-223°C wherein $R^6$ denotes H and $R^4$ denotes n-propyl;
- Compound of melting point 233°C wherein $R^6$ denotes cyclopentyl and $R^4$ denotes n-propyl;

optionally in the form of their racemates, the enantiomers, the diastereomers and the mixtures thereof and optionally the pharmacologically harmless acid addition salts thereof.

**5.** Compound obtainable according to claim 1 wherein $R^2$ denotes noradamantan-3-yl, selected from the group consisting of:

- Compound of melting point 325-327°C wherein $R^6$ denotes 4-fluorobenzyl and $R^4$ denotes ethyl;
- Compound of melting point 315-317°C wherein $R^6$ denotes 3,4-difluorobenzyl and $R^4$ denotes ethyl;
- Compound of melting point 320-322°C wherein $R^6$ denotes benzyl and $R^4$ denotes ethyl;
- Compound of melting point 330-332°C wherein $R^6$ denotes 3-pyridylmethyl and $R^4$ denotes ethyl;

optionally in the form of their racemates, the enantiomers, the diastereomers and the mixtures thereof and optionally the pharmacologically harmless acid addition salts thereof.

**6.** Compound obtainable according to claim 1, selected from the group consisting of:

- Compound of melting point 300°C (decomp.) wherein $R^6$ denotes n-propyl, $R^4$ denotes n-propyl and $R^2$ denotes phenyl;
- Compound of melting point 221°C wherein $R^6$ denotes n-propyl, $R^4$ denotes n-propyl and $R^2$ denotes 4-fluorobenzyl;
- Compound of melting point 248-250°C wherein $R^6$ denotes cyclopentyl, $R^4$ denotes n-propyl and $R^2$ denotes methyl;
- Compound of melting point 227°C wherein $R^6$ denotes benzyl, $R^4$ denotes ethyl and $R^2$ denotes 3-tetrahydrofuryl;
- Compound of melting point 237°C wherein $R^6$ denotes benzyl, $R^4$ denotes ethyl and $R^2$ denotes 4-tetrahydropyranyl;

- Compound of melting point 287°C wherein $R^6$ denotes benzyl, $R^4$ denotes ethyl and $R^2$ denotes 2-pyridyl;
- Compound of melting point 346°C wherein $R^6$ denotes benzyl, $R^4$ denotes ethyl and $R^2$ denotes 4-pyridyl;
- Compound of melting point 202°C wherein $R^6$ denotes benzyl, $R^4$ denotes n-propyl and $R^2$ denotes n-propyl;
- Compound of melting point 331-332°C wherein $R^6$ denotes benzyl, $R^4$ denotes ethyl and $R^2$ denotes 3-pyridyl;
- Compound of melting point 260-262°C wherein $R^6$ denotes benzyl, $R^4$ denotes ethyl and $R^2$ denotes cyclohexyl;
- Compound of melting point 385-387°C wherein $R^6$ denotes 2-furyl, $R^4$ denotes n-propyl and $R^2$ denotes 2-furyl;
- Compound of melting point 289-290°C wherein $R^6$ denotes cyclpentyl, $R^4$ denotes n-propyl and $R^2$ denotes 3-tetrahydrofuryl;
- Compound of melting point 262-264°C wherein $R^6$ denotes 4-fluorobenzyl, $R^4$ denotes ethyl and $R^2$ denotes tert.-butyl;
- Compound of melting point 337-339°C wherein $R^6$ denotes 4-fluorobenzyl, $R^4$ denotes ethyl and $R^2$ denotes adamantan-1-yl;

optionally in the form of their racemates, the enantiomers, the diastereomers and the mixtures thereof and optionally the pharmacologically harmless acid addition salts thereof.

**7.** Use of a compound according to one of claims 1 to 6 for preparing a pharmaceutical composition.

**8.** Pharmaceutical preparations containing as active substance one or more compounds according to one of claims 1 to 6 or the physiologically acceptable acid addition salts thereof combined with conventional excipients and/or carriers.

## Revendications

**1.** Composés pouvant être obtenus par les étapes réactionnelles suivantes:

a) conversion de 0,1 mole d'un triazole de formule générale (3)

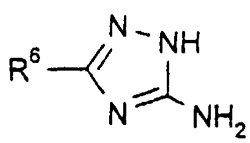

(3)

où
$R^6$ peut représenter l'hydrogène, méthyle, éthyle, n-propyle, i-propyle, n-butyle, tert.-butyle, phényle, benzyle, cyclopentyle, 2-furyle, 3-pyridylméthyle, cyclohexylméthyle, 2-phényléthyle, (4-Meo-Ph)-O-$CH_2$-, (4-MeO-Ph)-$CH_2$-O-$CH_2$-, (4-$PhCH_2O$-Ph)-$CH_2$-, 4-F-Ph-$CH_2$- ou 3,4-F-Ph-$CH_2$-, avec 0,13 mole de cyanoacétate d'éthyle en présence de 0,1 mole d'éthylate de sodium dans l'éthanol absolu à reflux pendant une durée de 4-6 h en la triazolopyrimidine correspondante, qui est isolée après refroidissement du mélange réactionnel, puis addition d'eau et acidification
b) conversion subséquente de 66 mmoles de la triazolopyrimidine ainsi obtenue à la température ambiante avec 76 mmoles d'un halogénure d'alkyle du type R'-X où R' représente le reste $R^4$, où
$R^4$ représente méthyle, éthyle, n-propyle, n-butyle, n-pentyle ou benzyle,
dans le diméthylformamide anhydre en présence de 76 mmoles de carbonate de potassium pendant une durée de 1-2 jours en la triazolopyrimidine alkylée correspondante, qui, après son isolement, est éventuellement soumise à une purification chromatographique,
c) conversion de 10 mmoles de l'alkyltriazolopyrimidine résultant de l'étape b) avec 20 mmoles de nitrite d'isoamyle à une température < 0°C dans le diméthylformamide anhydre pendant une durée de 4-24 h en le composé nitroso correspondant, qui est isolé,
d1) mise en suspension dans l'eau de 12,7 mmoles d'un dérivé nitroso obtenu selon l'étape c), addition de 4,9 g de $Na_2S_2O_4$, chauffage de la suspension à 80°C, addition de 15 ml de $H_2SO_4$ à 50% et chauffage du mélange à reflux jusqu'à la conversion totale, de sorte que, après refroidissement, le produit peut être isolé, directement sous forme d'hémisulfate qui précipite ou, après neutralisation avec de la lessive de soude à 30%,

sous forme de base libre précipitée,

ou

d2) dissolution de 11,5 mmoles d'un dérivé nitroso obtenu selon l'étape c) dans un mélange de 175 ml d'ammoniaque à 25% et de 36 ml d'éthanol et conversion avec une solution de 6,1 g de $Na_2S_2O_4$ dans 57 ml d'eau à la température ambiante pendant une durée de 1,5-3 h, séparation de la diamine résultante par filtration,
e) addition à 30 mmoles d'une diamine obtenue selon l'étape d1) ou selon l'étape d2), en suspension dans le diméthylformamide anhydre, de 45 mmoles de diméthylaminopyridine (de 78 mmoles dans le cas d'hémisulfates) et agitation du mélange pendant une durée de 30 minutes à la température ambiante, refroidissement du mélange réactionnel à une température de 5-10°C et addition de 39 mmoles d'un chlorure d'acide carboxylique de type $R^2$-COCl dissous dans 16 ml de diméthylformamide anhydre, où
$R^2$ représente méthyle, éthyle, n-propyle, tert-butyle, phényle, benzyle, 2-furyle, 2-pyridyle, 3-pyridyle, 4-pyridyle, 3-tétrahydrofuranyle, 4-tétrahydropyranyle, cyclopentyle, cyclohexyle, 4-F-Ph-$CH_2$-, adamantan-1-yle ou noradamantan-3-yle,
et agitation pendant 2 heures à 10°C puis agitation pendant une nuit à la température ambiante, d'où il résulte les amides correspondants qui sont isolés après traitement et purification par cristallisation ou chromatographie,
f) cyclisation de 14,4 mmoles des dérivés d'acide carboxylique obtenus dans l'étape e) par chauffage des amides dans un mélange de solvants constitué par 75,5 ml d'eau et 37,8 ml d'éthanol après addition de 17,4 ml de NaOH à 50% et de 4,82 g de $Ca(OH)_2$ pendant une durée de 24 h à la température de reflux et isolement des produits de cyclisation correspondants après refroidissement et acidification du mélange réactionnel et, éventuellement, purification par recristallisation ou chromatographie.

**2.** Composés pouvant être obtenus selon la revendication 1 où $R^2$ représente cyclopentyle, choisis dans le groupe consistant en:

- un composé de point de fusion de 250°C, où $R^6$ représente H et $R^4$ représente n-propyle;
- un composé de point de fusion de 262-264°C, où $R^6$ représente méthyle et $R^4$ représente n-propyle;
- un composé de point de fusion de 253°C, où $R^6$ représente éthyle et $R^4$ représente n-propyle;
- un composé de point de fusion de 256°C, où $R^6$ représente n-propyle et $R^4$ représente n-propyle;
- un composé de point de fusion de 260-261°C, où $R^6$ représente i-propyle et $R^4$ représente n-propyle;
- un composé de point de fusion de 246°C, où $R^6$ représente n-butyle et $R^4$ représente n-propyle;
- un composé de point de fusion de 310°C, où $R^6$ représente t-butyle et $R^4$ représente n-propyle;
- un composé de point de fusion > 300°C (déc.), où $R^6$ représente 2-furyle et $R^4$ représente n-propyle;
- un composé de point de fusion de 270°C, où $R^6$ représente benzyle et $R^4$ représente benzyle;
- un composé de point de fusion de 265-267°C, où $R^6$ représente benzyle et $R^4$ représente méthyle;
- un composé de point de fusion de 242°C, où $R^6$ représente benzyle et $R^4$ représente éthyle;
- un composé de point de fusion de 234-235°C, où $R^6$ représente benzyle et $R^4$ représente n-propyle;
- un composé de point de fusion de 238-240°C, où $R^6$ représente benzyle et $R^4$ représente n-butyle;
- un composé de point de fusion de 231-236°C, où $R^6$ représente benzyle et $R^4$ représente n-pentyle;
- un composé de point de fusion > 300°C (déc.), où $R^6$ représente phényle et $R^4$ représente n-propyle;
- un composé de point de fusion de 217-220°C, où $R^6$ représente H et $R^5$ représente n-propyle;
- un composé de point de fusion de 260°C, où $R^6$ représente H et $R^5$ représente éthyle;
- un composé de point de fusion de 261-263°C, où $R^6$ représente 3-pyridylméthyle et $R^4$ représente éthyle;
- un composé de point de fusion de 273-274°C, où $R^6$ représente cyclohexylméthyle et $R^4$ représente éthyle;
- un composé de point de fusion de 292-294°C, où $R^6$ représente 2-phényléthyle et $R^4$ représente éthyle;
- un composé de point de fusion de 306-308°C, où $R^6$ représente cyclopentyle et $R^4$ représente n-propyle;
- un composé de point de fusion de 234-236°C, où $R^6$ représente (4-MeO-Ph)-$OCH_2$- et $R^4$ représente éthyle;
- un composé de point de fusion de 182°C, où $R^6$ représente (4-MeO-Ph)-$CH_2$-O-$CH_2$- et $R^4$ représente éthyle;
- un composé de point de fusion de 251°C, où $R^6$ représente (4-$PhCH_2O$-Ph)-$CH_2$- et $R^4$ représente éthyle;
- un composé de point de fusion de 252-254°C, où $R^6$ représente 4-fluorobenzyle et $R^4$ représente éthyle;
- un composé de point de fusion de 264-266°C, où $R^6$ représente 3,4-difluorobenzyle et $R^4$ représente éthyle;

éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

**3.** Composés pouvant être obtenus selon la revendication 1 où $R^2$ représente éthyle, choisis dans le groupe consistant en:

- un composé de point de fusion de 229°C, où $R^6$ représente éthyle et $R^4$ représente n-propyle;
- un composé de point de fusion de 245-248°C, où $R^6$ représente cyclopentyle et $R^4$ représente n-propyle;
- un composé de point de fusion > 300°C (déc.), où $R^6$ représente 2-furyle et $R^4$ représente n-propyle;
- un composé de point de fusion de 223°C, où $R^6$ représente benzyle et $R^4$ représente n-propyle;
- un composé de point de fusion > 300°C (déc.), où $R^6$ représente phényle et $R^4$ représente n-propyle;
- un composé de point de fusion de 225-227°C, où $R^6$ représente 3-pyridylméthyle et $R^4$ représente éthyle;
- un composé de point de fusion de 230-232°C, où $R^6$ représente cyclohexylméthyle et $R^4$ représente éthyle;
- un composé de point de fusion de 282-283°C, où $R^6$ représente 2-phényléthyle et $R^4$ représente éthyle;
- un composé de point de fusion de 225°C, où $R^6$ représente (4-MeO-Ph)-$OCH_2$- et $R^4$ représente éthyle;

éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

**4.** Composés pouvant être obtenus selon la revendication 1 où $R^2$ représente benzyle, choisis dans le groupe consistant en:

- un composé de point de fusion de 306°C, où $R^6$ représente H et $R^4$ représente méthyle;
- un composé de point de fusion de 222-223°C, où $R^6$ représente H et $R^4$ représente n-propyle;
- un composé de point de fusion de 233°C, où $R^6$ représente cyclopentyle et $R^4$ représente n-propyle;

éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

**5.** Composés pouvant être obtenus selon la revendication 1 où $R^2$ représente noradamantan-3-yle, choisis dans le groupe consistant en:

- un composé de point de fusion de 325-327°C, où $R^6$ représente 4-fluorobenzyle et $R^4$ représente éthyle;
- un composé de point de fusion de 315-317°C, où $R^6$ représente 3,4-difluorobenzyle et $R^4$ représente éthyle;
- un composé de point de fusion de 320-322°C, où $R^6$ représente benzyle et $R^4$ représente éthyle;
- un composé de point de fusion de 330-332°C, où R6 représente 3-pyridylméthyle et $R^4$ représente éthyle; éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

**6.** Composés pouvant être obtenus selon la revendication 1 choisis dans le groupe consistant en:

- un composé de point de fusion de 300°C (déc.), où $R^6$ représente n-propyle, $R^4$ représente n-propyle, $R^2$ représente phényle;
- un composé de point de fusion de 221°C, où $R^6$ représente n-propyle, $R^4$ représente n-propyle, $R^2$ représente 4-fluorobenzyle;
- un composé de point de fusion de 248-250°C, où $R^6$ représente cyclopentyle, $R^4$ représente n-propyle, $R^2$ représente méthyle;
- un composé de point de fusion de 227°C, où $R^6$ représente benzyle, $R^4$ représente éthyle, $R^2$ représente 3-tétrahydrofuryle;
- un composé de point de fusion de 237°C, où $R^6$ représente benzyle, $R^4$ représente éthyle, $R^2$ représente 4-tétrahydropyranyle;
- un composé de point de fusion de 287°C, où $R^6$ représente benzyle, $R^4$ représente éthyle, $R^2$ représente 2-pyridyle;
- un composé de point de fusion de 346°C, où $R^6$ représente benzyle, $R^4$ représente éthyle, $R^2$ représente 4-pyridyle;
- un composé de point de fusion de 202°C, où $R^6$ représente benzyle, $R^4$ représente n-propyle, $R^2$ représente n-propyle;
- un composé de point de fusion de 331-332°C, où $R^6$ représente benzyle, $R^4$ représente éthyle, $R^2$ représente 3-pyridyle;
- un composé de point de fusion de 260-262°C, où $R^6$ représente benzyle, $R^4$ représente éthyle, $R^2$ représente cyclohexyle;

- un composé de point de fusion de 385-387°C, où $R^6$ représente 2-furyle, $R^4$ représente n-propyle, $R^2$ représente 2-furyle;
- un composé de point de fusion de 289-290°C, où $R^6$ représente cyclopentyle, $R^4$ représente n-propyle, $R^2$ représente 3-tétrahydrofuryle;
- un composé de point de fusion de 262-264°C, où $R^6$ représente 4-fluorobenzyle, $R^4$ représente éthyle, $R^2$ représente tert.-butyle;
- un composé de point de fusion de 337-339°C, où $R^6$ représente 4-fluorobenzyle, $R^4$ représente éthyle, $R^2$ représente adamantan-1-yle;

éventuellement sous forme de leurs racémates, de leurs énantiomères, sous forme de leurs diastéréoisomères et de leurs mélanges, ainsi éventuellement que de leurs sels d'addition d'acide pharmacologiquement acceptables.

**7.** Utilisation d'un composé selon l'une des revendications 1 à 6 pour la préparation d'un médicament.

**8.** Préparations pharmaceutiques contenant comme principe actif un ou plusieurs composés selon l'une des revendications 1 à 6 ou leurs sels d'addition d'acide physiologiquement acceptables en combinaison avec des adjuvants et/ou supports courants.